# EUROPEAN PATENT APPLICATION

(11) **EP 4 830 618 A1**
(43) Date of publication of application: **28.10.2026**
(21) Application number: 25741468.0
(22) Date of filing: 13.01.2025
(51) Int. Cl.: A61B 5/00, A61B 5/0205, A61B 5/11, G01D 21/02

(54) **SLEEP DETECTION METHOD, RELATED APPARATUS, AND COMMUNICATION SYSTEM**

(30) Priority: 15.01.2024 CN 202410063004
(71) Applicant: Huawei Technologies Co., Ltd., Shenzhen, Guangdong 518129 (CN)
(72) Inventor: PENG, Wei, Shenzhen, Guangdong 518129 (CN); CAI, Tingting, Shenzhen, Guangdong 518129 (CN); YU, Bing, Shenzhen, Guangdong 518129 (CN)
(74) Representative: Goddar, Heinz J.
(86) International application number: PCT/CN2025/072122
(87) International publication number: WO 2025/152907

(57) **Abstract**

This application provides a sleep monitoring method, a related apparatus, and a communication system. In the method, fall-asleep time of a user is determined with reference to data obtained through a plurality of types of detection, such as heart rate detection, activity amount statistics collection, get-on-bed action detection, walking feature detection, ambient light detection, ambient sound detection, and usage detection of an electronic device like a mobile phone and/or a tablet computer, thereby improving accuracy of detecting the fall-asleep time. In addition, in the method, get-on-bed time and get-off-bed time of the user are further determined with reference to heart rate data, activity amount data, and a get-on-bed/get-off-bed walking feature and action feature.

## Description

This application claims priority to Chinese Patent Application No. 202410063004.8, filed with the China National Intellectual Property Administration on January 15, 2024 and entitled "SLEEP MONITORING METHOD, RELATED APPARATUS, AND COMMUNICATION SYSTEM", which is incorporated herein by reference in its entirety.

### TECHNICAL FIELD

This application relates to the field of terminal technologies, and in particular, to a sleep monitoring method, a related apparatus, and a communication system.

### BACKGROUND

With the development of electronic devices and research on users' sleep status, more electronic devices (such as a smartwatch and a smart band) can monitor users' sleep status. For example, an electronic device may detect fall-asleep time, wake-up time, and sleep status stages of a user. However, the electronic device usually performs sleep monitoring based on heart rate data and motion data of the user, and a detection result often has an error. For example, in a scenario where the user lies quietly on bed and uses a mobile phone, or listens to music, or watches television, or the like, the heart rate data of the user is usually low, and an activity amount of the user is low. The electronic device may determine, when the user has not actually fallen asleep, that the user has fallen asleep.

### SUMMARY

This application provides a sleep monitoring method, a related apparatus, and a communication system. In the method, fall-asleep time of a user is determined with reference to data obtained through a plurality of types of detection, such as heart rate detection, activity amount statistics collection, get-on-bed action detection, walking feature detection, ambient light detection, ambient sound detection, and usage detection of an electronic device like a mobile phone and/or a tablet computer, thereby improving accuracy of detecting the fall-asleep time.

According to a first aspect, this application provides a sleep monitoring method. Specifically, the method includes: obtaining a heart rate and motion data of a user, where the motion data includes an acceleration and/or an angular velocity; obtaining ambient light brightness, an ambient sound volume, and usage data of one or more electronic devices, where the usage data of the one or more electronic devices includes screen-off time of the one or more electronic devices; determining an activity amount, a walking feature, and a get-on-bed action feature based on the motion data; and determining fall-asleep time of the user based on the heart rate, the activity amount, the walking feature, the get-on-bed action feature, the ambient light brightness, the ambient sound volume, and the usage data of the one or more electronic devices.

The one or more electronic devices may include one or more of the following: a mobile phone, a tablet computer, a television, and a notebook computer.

It can be learned that, in the foregoing method, a plurality of factors, namely, the heart rate, motion data, ambient light brightness, ambient sound volume, and usage data of the one or more electronic devices are comprehensively considered, to determine the fall-asleep time of the user, thereby improving accuracy of detecting the fall-asleep time.

With reference to the first aspect, in some embodiments, determining a first fall-asleep time point based on the get-on-bed action feature, determining a second fall-asleep time point based on the activity amount, determining a third fall-asleep time point based on the walking feature, determining a fourth fall-asleep time point based on the heart rate, determining a fifth fall-asleep time point based on the ambient light brightness, determining a sixth fall-asleep time point based on the ambient sound volume, and determining a seventh fall-asleep time point based on the usage data of the one or more electronic devices; and determining the fall-asleep time of the user based on the first fall-asleep time point, the second fall-asleep time point, the third fall-asleep time point, the fourth fall-asleep time point, the fifth fall-asleep time point, the sixth fall-asleep time point, and the seventh fall-asleep time point.

The first fall-asleep time point is time at which a get-on-bed action occurs, the second fall-asleep time point is time at which the activity amount is less than an activity amount threshold, the third fall-asleep time point is time at which the user changes from a walking state to a non-walking state, the fifth fall-asleep time point is time at which the ambient light brightness is less than a brightness threshold, the sixth fall-asleep time point is time at which the ambient sound volume is less than a volume threshold, and the seventh fall-asleep time point is time at which the one or more electronic devices become off-screen.

With reference to the first aspect, in some embodiments, determining first time based on the second fall-asleep time point and the fourth fall-asleep time point; and when the first fall-asleep time point, the third fall-asleep time point, the fifth fall-asleep time point, the sixth fall-asleep time point, and the seventh fall-asleep time point are all earlier than the first time, determining the first time as the fall-asleep time of the user.

The first time is any time between the second fall-asleep time point and the fourth fall-asleep time point, or an average value of the second fall-asleep time point and the fourth fall-asleep time point.

It can be learned that, the mobile phone can determine the fall-asleep time of the user by using, as a reference, the second fall-asleep time point determined based on the activity amount and the fourth fall-asleep time point determined based on the heart rate, and using the first fall-asleep time point, the third fall-asleep time point, and the fifth fall-asleep time point to the seventh fall-asleep time point as auxiliary. It may be understood that, the activity amount and the heart rate can directly reflect whether the user falls asleep. The get-on-bed action, walking feature, ambient light brightness, ambient sound volume, and screen-off of the mobile phone may reflect that the user is preparing to fall asleep. The user may need a period of time from preparing to fall asleep to actually entering a sleep state. The first fall-asleep time point and the third fall-asleep time point are earlier than the first time, which may indicate that the user falls asleep after a period of time after getting on bed. The fifth fall-asleep time point is earlier than the first time, which may indicate that the ambient light becomes dark before the user falls asleep. The sixth fall-asleep time point is earlier than the first time, which may indicate that an ambient sound volume decreases before the user falls asleep. The seventh fall-asleep time point is earlier than the first time, which may indicate that the screen of the electronic device like the mobile phone is turned off before the user falls asleep.

With reference to the first aspect, in some embodiments, the method further includes: when both the fifth fall-asleep time point and the sixth fall-asleep time point are earlier than the seventh fall-asleep time point, and the seventh fall-asleep time point is later than the first time, determining second time based on the seventh fall-asleep time point, and determining the second time as the fall-asleep time of the user, where the second time is later than the seventh fall-asleep time point.

It can be learned that, when it is detected, based on the activity amount and the heart rate, that the user falls asleep, the user may be in a state of lying quietly on bed and using the mobile phone, that is, the user actually does not fall asleep. In addition, after the user turns off the screen of the mobile phone, the user usually needs a period of time before falling asleep. Therefore, the second time after a screen of the electronic device like the mobile phone is turned off may be used as the fall-asleep time of the user. Sleep monitoring is performed with reference to the usage of the electronic device like the mobile phone, thereby improving accuracy of detecting the fall-asleep time.

With reference to the first aspect, in some embodiments, the method further includes: when both the sixth fall-asleep time point and the seventh fall-asleep time point are earlier than the fifth fall-asleep time point, the fifth fall-asleep time point is later than the first time, and a time difference between the fifth fall-asleep time point and the first time is less than or equal to a first difference, determining third time based on the fifth fall-asleep time point, and determining the third time as the fall-asleep time of the user, where the third time is later than the fifth fall-asleep time point.

It can be learned that, when both the sixth fall-asleep time point and the seventh fall-asleep time point are earlier than the fifth fall-asleep time point, the user may first turn off audio that is being played in a sleep environment and turn off the screen of the electronic device like the mobile phone before turning off a light to sleep. When the fifth fall-asleep time point is later than the first time, and the time difference between the fifth fall-asleep time point and the first time is less than or equal to the first difference, the user may turn off a light and prepare to fall asleep after lying quietly on bed for a period of time in a light-on state. Therefore, the actual fall-asleep time of the user may be slightly later than time when the ambient light brightness becomes dark. Sleep monitoring is performed with reference to the ambient light brightness, thereby improving accuracy of detecting the fall-asleep time.

With reference to the first aspect, in some embodiments, the method further includes: when both the fifth fall-asleep time point and the seventh fall-asleep time point are earlier than the sixth fall-asleep time point, the sixth fall-asleep time point is later than the first time, and a time difference between the sixth fall-asleep time point and the first time is less than or equal to a second difference, determining fourth time based on the sixth fall-asleep time point, and determining the fourth time as the fall-asleep time of the user, where the fourth time is later than the sixth fall-asleep time point.

It can be learned that, when both the fifth fall-asleep time point and the seventh fall-asleep time point are earlier than the sixth fall-asleep time point, the user may first turn off the screen of the mobile phone and turn off the light before the audio that is being played is turned off. When the sixth fall-asleep time point is later than the first time, and the time difference between the sixth fall-asleep time point and the first time is less than or equal to the second difference, the user may actively turn off the played audio after lying quietly on bed and listening to music for a period of time. Therefore, the actual fall-asleep time of the user may be slightly later than the sixth fall-asleep time point (namely, time when the ambient sound volume decreases).

With reference to the first aspect, in some embodiments, the method further includes: when the sixth fall-asleep time point is later than the first time, the time difference between the sixth fall-asleep time point and the first time is greater than the second difference, and both the fifth fall-asleep time point and the seventh fall-asleep time point are earlier than the first time, determining fifth time based on the first time, and determining the fifth time as the fall-asleep time of the user, where the fifth time is later than the first time.

With reference to the first aspect, in some embodiments, the method further includes: when the sixth fall-asleep time point and the seventh fall-asleep time point are later than the first time, the time difference between the sixth fall-asleep time point and the first time is greater than the second difference, and the fifth fall-asleep time point is earlier than the first time, determining sixth time based on the seventh fall-asleep time point, and determining the sixth time as the fall-asleep time of the user, where the sixth time is later than the seventh fall-asleep time point.

With reference to the first aspect, in some embodiments, the method further includes: when the fifth fall-asleep time point and the sixth fall-asleep time point are later than the first time, the time difference between the fifth fall-asleep time point and the first time is less than or equal to the first difference, the time difference between the sixth fall-asleep time point and the first time is greater than the second difference, and the seventh fall-asleep time point is earlier than the first time, determining seventh time based on the fifth fall-asleep time point, and determining the seventh time as the fall-asleep time of the user, where the seventh time is later than the fifth fall-asleep time point.

With reference to the first aspect, in some embodiments, the method further includes: when the fifth fall-asleep time point and the sixth fall-asleep time point are later than the first time, the time difference between the fifth fall-asleep time point and the first time is greater than the first difference, the time difference between the sixth fall-asleep time point and the first time is greater than the second difference, and the seventh fall-asleep time point is earlier than the first time, determining eighth time based on the first time, and determining the eighth time as the fall-asleep time of the user, where the eighth time is later than the first time.

It can be learned that, when the sixth fall-asleep time point is later than the first time and the time difference between the sixth fall-asleep time point and the first time is greater than the second difference, the user may fall asleep in an audio playing process. The audio continues to be played and is not paused after the user falls asleep. A moment (namely, the sixth fall-asleep time point) when the ambient sound volume decreases cannot reflect the actual fall-asleep time of the user. If the fifth fall-asleep time point and/or the seventh fall-asleep time point are/is within a time period from the first time to the sixth fall-asleep time point, the user may actively turn off the light after lying quietly on bed for a period of time and/or actively turn off the screen of the mobile phone after lying quietly on bed and using the mobile phone for a period of time, and continue to listen to the audio. Therefore, when the moment (namely, the sixth fall-asleep time point) at which the ambient sound volume decreases cannot reflect the actual fall-asleep time of the user, the mobile phone may determine the fall-asleep time point based on the fifth fall-asleep time point and/or the seventh fall-asleep time point. In the foregoing embodiment, sleep monitoring is performed with reference to the ambient light brightness, ambient sound volume, and usage of the mobile phone, thereby improving accuracy of detecting the fall-asleep time.

With reference to the first aspect, in some embodiments, the method further includes: determining a first time period based on the fall-asleep time of the user, and obtaining a first heart rate and first motion data of the user in the first time period; determining a second time period based on wake-up time of the user, and obtaining a second heart rate and second motion data of the user in the second time period; determining a first activity amount, a first walking feature, and a first get-on-bed action feature based on the first motion data, and determining a second activity amount, a second walking feature, and a first get-off-bed action feature based on the second motion data; and determining get-on-bed time and get-off-bed time of the user based on the first heart rate, the second heart rate, the first activity amount, the first walking feature, the first get-on-bed action feature, the second activity amount, the second walking feature, and the first get-off-bed action feature.

It can be learned that, in the foregoing method, the get-on-bed time and get-off-bed time of the user are determined with reference to detection of walking features, heart rates, activity amounts, and a get-on-bed/get-off-bed action near the fall-asleep time and the wake-up time. Walking behaviors obviously change when the user gets on bed and gets off bed, and walking features in a get-on-bed phase and a get-off-bed phase are symmetrical. Therefore, the sleep monitoring method performed with reference to the walking features can improve accuracy of detecting the get-on-bed/get-off-bed time.

With reference to the first aspect, in some embodiments, determining a first get-on-bed time point based on the first heart rate and the first activity amount; determining a first get-off-bed time point based on the second heart rate and the second activity amount; determining a second get-on-bed time point and a second get-off-bed time point based on the first walking feature and the second walking feature; determining a third get-on-bed time point based on the first get-on-bed action feature; determining a third get-off-bed time point based on the first get-off-bed action feature; determining the get-on-bed time of the user based on the first get-on-bed time point, the second get-on-bed time point, and the third get-on-bed time point; and determining the get-off-bed time of the user based on the first get-off-bed time point, the second get-off-bed time point, and the third get-off-bed time point.

The first get-on-bed time point is time at which the first heart rate decreases to a first heart rate threshold and the first activity amount decreases to a first activity amount threshold, and the first get-off-bed time point is time at which the second heart rate increases to a second heart rate threshold and/or the second activity amount increases to a second activity amount threshold; the second get-on-bed time point is time at which a last walking behavior occurs in the first time period, and the second get-off-bed time point is time at which a first walking behavior occurs in the second time period; and the third get-on-bed time point is time at which the get-on-bed action occurs, and the third get-off-bed time point is time at which a get-off-bed action occurs.

With reference to the first aspect, in some embodiments, when a time difference between any two of the first get-on-bed time point, the second get-on-bed time point, and the third get-on-bed time point is less than a third difference, determining, as the get-on-bed time of the user, an average value of the first get-on-bed time point, the second get-on-bed time point, and the third get-on-bed time point, or determining, as the get-on-bed time of the user, any time between earliest time and latest time in the first get-on-bed time point, the second get-on-bed time point, and the third get-on-bed time point; and when a time difference between any two of the first get-off-bed time point, the second get-off-bed time point, and the third get-off-bed time point is less than a fourth difference, determining, as the get-off-bed time of the user, an average value of the first get-off-bed time point, the second get-off-bed time point, and the third get-off-bed time point, or determining, as the get-off-bed time of the user, any time between earliest time and latest time in the first get-off-bed time point, the second get-off-bed time point, and the third get-off-bed time point.

With reference to the first aspect, in some embodiments, when a time difference between the second get-on-bed time point and the third get-on-bed time point is less than the third difference, and a time difference between the first get-on-bed time point and the second get-on-bed time point is greater than or equal to the third difference, determining, as the get-on-bed time of the user, an average value of the second get-on-bed time point and the third get-on-bed time point, or determining, as the get-on-bed time of the user, any time between the second get-on-bed time point and the third get-on-bed time point; and when a time difference between the second get-off-bed time point and the third get-off-bed time point is less than the fourth difference, and a time difference between the first get-off-bed time point and the second get-off-bed time point is greater than or equal to the fourth difference, determining, as the get-off-bed time of the user, an average value of the second get-off-bed time point and the third get-off-bed time point, or determining, as the get-off-bed time of the user, any time between the second get-off-bed time point and the third get-off-bed time point.

The first time period includes a time period before the fall-asleep time of the user, and the second time period includes a time period after the wake-up time of the user.

With reference to the first aspect, in some embodiments, the method further includes: displaying the fall-asleep time, wake-up time, get-on-bed time, and get-off-bed time of the user.

With reference to the first aspect, in some embodiments, the heart rate and motion data of the user are obtained by a wearable device, and the wearable device includes one or more of the following: a smartwatch and a smart band.

According to a second aspect, this application provides a sleep monitoring method. The method may be applied to a communication system including a smartwatch and a mobile phone. The smartwatch may obtain a heart rate and motion data of a user, where the motion data includes an acceleration and/or an angular velocity; the smartwatch may further obtain ambient light brightness, and an ambient sound volume; the mobile phone may obtain usage data of one or more electronic devices, where the usage data of the one or more electronic devices includes screen-off time of the one or more electronic devices; the mobile phone may determine an activity amount, a walking feature, and a get-on-bed action feature based on the motion data; and the mobile phone may determine fall-asleep time of the user based on the heart rate, the activity amount, the walking feature, the get-on-bed action feature, the ambient light brightness, the ambient sound volume, and the usage data of the one or more electronic devices.

The one or more electronic devices may include one or more of the following: a mobile phone, a tablet computer, a television, and a notebook computer.

In some embodiments, the smartwatch may send the heart rate and the motion data of the user to the mobile phone. The smartwatch may further send the ambient light brightness and the ambient sound volume to the mobile phone.

It can be learned that, in the foregoing method, a plurality of factors, namely, the heart rate, motion data, ambient light brightness, ambient sound volume, and usage data of the one or more electronic devices are comprehensively considered, to determine the fall-asleep time of the user, thereby improving accuracy of detecting the fall-asleep time.

With reference to the second aspect, in some embodiments, the mobile phone may determine a first fall-asleep time point based on the get-on-bed action feature, the mobile phone may determine a second fall-asleep time point based on the activity amount, the mobile phone may determine a third fall-asleep time point based on the walking feature, the mobile phone may determine a fourth fall-asleep time point based on the heart rate, the mobile phone may determine a fifth fall-asleep time point based on the ambient light brightness, the mobile phone may determine a sixth fall-asleep time point based on the ambient sound volume, and the mobile phone may determine a seventh fall-asleep time point based on the usage data of the one or more electronic devices; and the mobile phone may determine the fall-asleep time of the user based on the first fall-asleep time point, the second fall-asleep time point, the third fall-asleep time point, the fourth fall-asleep time point, the fifth fall-asleep time point, the sixth fall-asleep time point, and the seventh fall-asleep time point.

The first fall-asleep time point is time at which a get-on-bed action occurs, the second fall-asleep time point is time at which the activity amount is less than an activity amount threshold, the third fall-asleep time point is time at which the user changes from a walking state to a non-walking state, the fifth fall-asleep time point is time at which the ambient light brightness is less than a brightness threshold, the sixth fall-asleep time point is time at which the ambient sound volume is less than a volume threshold, and the seventh fall-asleep time point is time at which the one or more electronic devices become off-screen.

Optionally, one or more of the first fall-asleep time point to the sixth fall-asleep time point may be determined by the smartwatch. The smartwatch may send the fall-asleep time point determined by the smartwatch to the mobile phone.

With reference to the second aspect, in some embodiments, the mobile phone may determine first time based on the second fall-asleep time point and the fourth fall-asleep time point; and when the first fall-asleep time point, the third fall-asleep time point, the fifth fall-asleep time point, the sixth fall-asleep time point, and the seventh fall-asleep time point are all earlier than the first time, the mobile phone may determine the first time as the fall-asleep time of the user.

The first time is any time between the second fall-asleep time point and the fourth fall-asleep time point, or an average value of the second fall-asleep time point and the fourth fall-asleep time point.

It can be learned that, the mobile phone can determine the fall-asleep time of the user by using, as a reference, the second fall-asleep time point determined based on the activity amount and the fourth fall-asleep time point determined based on the heart rate, and using the first fall-asleep time point, the third fall-asleep time point, and the fifth fall-asleep time point to the seventh fall-asleep time point as auxiliary. It may be understood that, the activity amount and the heart rate can directly reflect whether the user falls asleep. The get-on-bed action, walking feature, ambient light brightness, ambient sound volume, and screen-off of the mobile phone may reflect that the user is preparing to fall asleep. The user may need a period of time from preparing to fall asleep to actually entering a sleep state. The first fall-asleep time point and the third fall-asleep time point are earlier than the first time, which may indicate that the user falls asleep after a period of time after getting on bed. The fifth fall-asleep time point is earlier than the first time, which may indicate that the ambient light becomes dark before the user falls asleep. The sixth fall-asleep time point is earlier than the first time, which may indicate that an ambient sound volume decreases before the user falls asleep. The seventh fall-asleep time point is earlier than the first time, which may indicate that the screen of the electronic device like the mobile phone is turned off before the user falls asleep.

With reference to the second aspect, in some embodiments, when both the fifth fall-asleep time point and the sixth fall-asleep time point are earlier than the seventh fall-asleep time point, and the seventh fall-asleep time point is later than the first time, the mobile phone may determine second time based on the seventh fall-asleep time point, and determine the second time as the fall-asleep time of the user, where the second time is later than the seventh fall-asleep time point.

It can be learned that, when it is detected, based on the activity amount and the heart rate, that the user falls asleep, the user may be in a state of lying quietly on bed and using the mobile phone, that is, the user actually does not fall asleep. In addition, after the user turns off the screen of the mobile phone, the user usually needs a period of time before falling asleep. Therefore, the second time after a screen of the electronic device like the mobile phone is turned off may be used as the fall-asleep time of the user. Sleep monitoring is performed with reference to the usage of the electronic device like the mobile phone, thereby improving accuracy of detecting the fall-asleep time.

With reference to the second aspect, in some embodiments, when both the sixth fall-asleep time point and the seventh fall-asleep time point are earlier than the fifth fall-asleep time point, the fifth fall-asleep time point is later than the first time, and a time difference between the fifth fall-asleep time point and the first time is less than or equal to a first difference, the mobile phone may determine third time based on the fifth fall-asleep time point, and determine the third time as the fall-asleep time of the user, where the third time is later than the fifth fall-asleep time point.

It can be learned that, when both the sixth fall-asleep time point and the seventh fall-asleep time point are earlier than the fifth fall-asleep time point, the user may first turn off audio that is being played in a sleep environment and turn off the screen of the electronic device like the mobile phone before turning off a light to sleep. When the fifth fall-asleep time point is later than the first time, and the time difference between the fifth fall-asleep time point and the first time is less than or equal to the first difference, the user may turn off a light and prepare to fall asleep after lying quietly on bed for a period of time in a light-on state. Therefore, the actual fall-asleep time of the user may be slightly later than time when the ambient light brightness becomes dark. Sleep monitoring is performed with reference to the ambient light brightness, thereby improving accuracy of detecting the fall-asleep time.

With reference to the second aspect, in some embodiments, when both the fifth fall-asleep time point and the seventh fall-asleep time point are earlier than the sixth fall-asleep time point, the sixth fall-asleep time point is later than the first time, and a time difference between the sixth fall-asleep time point and the first time is less than or equal to a second difference, the mobile phone may determine fourth time based on the sixth fall-asleep time point, and determine the fourth time as the fall-asleep time of the user, where the fourth time is later than the sixth fall-asleep time point.

It can be learned that, when both the fifth fall-asleep time point and the seventh fall-asleep time point are earlier than the sixth fall-asleep time point, the user may first turn off the screen of the mobile phone and turn off the light before the audio that is being played is turned off. When the sixth fall-asleep time point is later than the first time, and the time difference between the sixth fall-asleep time point and the first time is less than or equal to the second difference, the user may actively turn off the played audio after lying quietly on bed and listening to music for a period of time. Therefore, the actual fall-asleep time of the user may be slightly later than the sixth fall-asleep time point (namely, time when the ambient sound volume decreases).

With reference to the second aspect, in some embodiments, when the sixth fall-asleep time point is later than the first time, the time difference between the sixth fall-asleep time point and the first time is greater than the second difference, and both the fifth fall-asleep time point and the seventh fall-asleep time point are earlier than the first time, the mobile phone may determine fifth time based on the first time, and determine the fifth time as the fall-asleep time of the user, where the fifth time is later than the first time.

With reference to the second aspect, in some embodiments, when the sixth fall-asleep time point and the seventh fall-asleep time point are later than the first time, the time difference between the sixth fall-asleep time point and the first time is greater than the second difference, and the fifth fall-asleep time point is earlier than the first time, the mobile phone may determine sixth time based on the seventh fall-asleep time point, and determine the sixth time as the fall-asleep time of the user, where the sixth time is later than the seventh fall-asleep time point.

With reference to the second aspect, in some embodiments, when the fifth fall-asleep time point and the sixth fall-asleep time point are later than the first time, the time difference between the fifth fall-asleep time point and the first time is less than or equal to the first difference, the time difference between the sixth fall-asleep time point and the first time is greater than the second difference, and the seventh fall-asleep time point is earlier than the first time, the mobile phone may determine seventh time based on the fifth fall-asleep time point, and determine the seventh time as the fall-asleep time of the user, where the seventh time is later than the fifth fall-asleep time point.

With reference to the second aspect, in some embodiments, when the fifth fall-asleep time point and the sixth fall-asleep time point are later than the first time, the time difference between the fifth fall-asleep time point and the first time is greater than the first difference, the time difference between the sixth fall-asleep time point and the first time is greater than the second difference, and the seventh fall-asleep time point is earlier than the first time, the mobile phone may determine eighth time based on the first time, and determine the eighth time as the fall-asleep time of the user, where the eighth time is later than the first time.

It can be learned that, when the sixth fall-asleep time point is later than the first time and the time difference between the sixth fall-asleep time point and the first time is greater than the second difference, the user may fall asleep in an audio playing process. The audio continues to be played and is not paused after the user falls asleep. A moment (namely, the sixth fall-asleep time point) when the ambient sound volume decreases cannot reflect the actual fall-asleep time of the user. If the fifth fall-asleep time point and/or the seventh fall-asleep time point are/is within a time period from the first time to the sixth fall-asleep time point, the user may actively turn off the light after lying quietly on bed for a period of time and/or actively turn off the screen of the mobile phone after lying quietly on bed and using the mobile phone for a period of time, and continue to listen to the audio. Therefore, when the moment (namely, the sixth fall-asleep time point) at which the ambient sound volume decreases cannot reflect the actual fall-asleep time of the user, the mobile phone may determine the fall-asleep time point based on the fifth fall-asleep time point and/or the seventh fall-asleep time point. In the foregoing embodiment, sleep monitoring is performed with reference to the ambient light brightness, ambient sound volume, and usage of the mobile phone, thereby improving accuracy of detecting the fall-asleep time.

With reference to the second aspect, in some embodiments, the mobile phone may determine a first time period based on the fall-asleep time of the user, and obtain a first heart rate and first motion data of the user in the first time period; the mobile phone may determine a second time period based on wake-up time of the user, and obtain a second heart rate and second motion data of the user in the second time period; the mobile phone may determine a first activity amount, a first walking feature, and a first get-on-bed action feature based on the first motion data, and determine a second activity amount, a second walking feature, and a first get-off-bed action feature based on the second motion data; and the mobile phone may determine get-on-bed time and get-off-bed time of the user based on the first heart rate, the second heart rate, the first activity amount, the first walking feature, the first get-on-bed action feature, the second activity amount, the second walking feature, and the first get-off-bed action feature.

The first heart rate, the second motion data, the second heart rate, and the second motion data may be obtained by the mobile phone from the smartwatch.

It can be learned that, in the foregoing method, the get-on-bed time and get-off-bed time of the user are determined with reference to detection of walking features, heart rates, activity amounts, and a get-on-bed/get-off-bed action near the fall-asleep time and the wake-up time. Walking behaviors obviously change when the user gets on bed and gets off bed, and walking features in a get-on-bed phase and a get-off-bed phase are symmetrical. Therefore, the sleep monitoring method performed with reference to the walking features can improve accuracy of detecting the get-on-bed/get-off-bed time.

With reference to the second aspect, in some embodiments, the mobile phone may determine a first get-on-bed time point based on the first heart rate and the first activity amount; the mobile phone may determine a first get-off-bed time point based on the second heart rate and the second activity amount; the mobile phone may determine a second get-on-bed time point and a second get-off-bed time point based on the first walking feature and the second walking feature; the mobile phone may determine a third get-on-bed time point based on the first get-on-bed action feature; the mobile phone may determine a third get-off-bed time point based on the first get-off-bed action feature; the mobile phone may determine the get-on-bed time of the user based on the first get-on-bed time point, the second get-on-bed time point, and the third get-on-bed time point; and the mobile phone may determine the get-off-bed time of the user based on the first get-off-bed time point, the second get-off-bed time point, and the third get-off-bed time point.

The first get-on-bed time point is time at which the first heart rate decreases to a first heart rate threshold and the first activity amount decreases to a first activity amount threshold, and the first get-off-bed time point is time at which the second heart rate increases to a second heart rate threshold and/or the second activity amount increases to a second activity amount threshold; the second get-on-bed time point is time at which a last walking behavior occurs in the first time period, and the second get-off-bed time point is time at which a first walking behavior occurs in the second time period; and the third get-on-bed time point is time at which the get-on-bed action occurs, and the third get-off-bed time point is time at which a get-off-bed action occurs.

With reference to the second aspect, in some embodiments, when a time difference between any two of the first get-on-bed time point, the second get-on-bed time point, and the third get-on-bed time point is less than a third difference, the mobile phone may determine, as the get-on-bed time of the user, an average value of the first get-on-bed time point, the second get-on-bed time point, and the third get-on-bed time point, or determine, as the get-on-bed time of the user, any time between earliest time and latest time in the first get-on-bed time point, the second get-on-bed time point, and the third get-on-bed time point; and when a time difference between any two of the first get-off-bed time point, the second get-off-bed time point, and the third get-off-bed time point is less than a fourth difference, the mobile phone may determine, as the get-off-bed time of the user, an average value of the first get-off-bed time point, the second get-off-bed time point, and the third get-off-bed time point, or determine, as the get-off-bed time of the user, any time between earliest time and latest time in the first get-off-bed time point, the second get-off-bed time point, and the third get-off-bed time point.

With reference to the second aspect, in some embodiments, when a time difference between the second get-on-bed time point and the third get-on-bed time point is less than the third difference, and a time difference between the first get-on-bed time point and the second get-on-bed time point is greater than or equal to the third difference, the mobile phone may determine, as the get-on-bed time of the user, an average value of the second get-on-bed time point and the third get-on-bed time point, or determine, as the get-on-bed time of the user, any time between the second get-on-bed time point and the third get-on-bed time point; and when a time difference between the second get-off-bed time point and the third get-off-bed time point is less than the fourth difference, and a time difference between the first get-off-bed time point and the second get-off-bed time point is greater than or equal to the fourth difference, the mobile phone may determine, as the get-off-bed time of the user, an average value of the second get-off-bed time point and the third get-off-bed time point, or determine, as the get-off-bed time of the user, any time between the second get-off-bed time point and the third get-off-bed time point.

The first time period includes a time period before the fall-asleep time of the user, and the second time period includes a time period after the wake-up time of the user.

With reference to the second aspect, in some embodiments, the smartwatch and/or the mobile phone may display the fall-asleep time, wake-up time, get-on-bed time, and get-off-bed time of the user.

According to a third aspect, this application provides a sleep monitoring method. The method may be applied to a communication system including a processing apparatus and a data collection apparatus. The data collection apparatus may obtain a heart rate and motion data of a user, where the motion data includes an acceleration and/or an angular velocity; the data collection apparatus may obtain ambient light brightness, an ambient sound volume, and usage data of one or more electronic devices, where the usage data of the one or more electronic devices includes screen-off time of the one or more electronic devices; and the processing apparatus may determine an activity amount, a walking feature, and a get-on-bed action feature based on the motion data, and determine fall-asleep time of the user based on the heart rate, the activity amount, the walking feature, the get-on-bed action feature, the ambient light brightness, the ambient sound volume, and the usage data of the one or more electronic devices.

The one or more electronic devices may include one or more of the following: a mobile phone, a tablet computer, a television, and a notebook computer.

It can be learned that, in the foregoing method, a plurality of factors, namely, the heart rate, motion data, ambient light brightness, ambient sound volume, and usage data of the one or more electronic devices are comprehensively considered, to determine the fall-asleep time of the user, thereby improving accuracy of detecting the fall-asleep time.

With reference to the third aspect, in some embodiments, the processing apparatus may determine a first fall-asleep time point based on the get-on-bed action feature, the processing apparatus may determine a second fall-asleep time point based on the activity amount, the processing apparatus may determine a third fall-asleep time point based on the walking feature and determine a fourth fall-asleep time point based on the heart rate, the processing apparatus may determine a fifth fall-asleep time point based on the ambient light brightness, the processing apparatus may determine a sixth fall-asleep time point based on the ambient sound volume, and the processing apparatus may determine a seventh fall-asleep time point based on the usage data of the one or more electronic devices; and the processing apparatus may determine the fall-asleep time of the user based on the first fall-asleep time point, the second fall-asleep time point, the third fall-asleep time point, the fourth fall-asleep time point, the fifth fall-asleep time point, the sixth fall-asleep time point, and the seventh fall-asleep time point.

The first fall-asleep time point is time at which a get-on-bed action occurs, the second fall-asleep time point is time at which the activity amount is less than an activity amount threshold, the third fall-asleep time point is time at which the user changes from a walking state to a non-walking state, the fifth fall-asleep time point is time at which the ambient light brightness is less than a brightness threshold, the sixth fall-asleep time point is time at which the ambient sound volume is less than a volume threshold, and the seventh fall-asleep time point is time at which the one or more electronic devices become off-screen.

With reference to the third aspect, in some embodiments, the processing apparatus may determine first time based on the second fall-asleep time point and the fourth fall-asleep time point; and when the first fall-asleep time point, the third fall-asleep time point, the fifth fall-asleep time point, the sixth fall-asleep time point, and the seventh fall-asleep time point are all earlier than the first time, the processing apparatus may determine the first time as the fall-asleep time of the user.

The first time is any time between the second fall-asleep time point and the fourth fall-asleep time point, or an average value of the second fall-asleep time point and the fourth fall-asleep time point.

It can be learned that, in the foregoing embodiment, the fall-asleep time of the user can be determined by using, as a reference, the second fall-asleep time point determined based on the activity amount and the fourth fall-asleep time point determined based on the heart rate, and using the first fall-asleep time point, the third fall-asleep time point, and the fifth fall-asleep time point to the seventh fall-asleep time point as auxiliary. It may be understood that, the activity amount and the heart rate can directly reflect whether the user falls asleep. The get-on-bed action, walking feature, ambient light brightness, ambient sound volume, and screen-off of the mobile phone may reflect that the user is preparing to fall asleep. The user may need a period of time from preparing to fall asleep to actually entering a sleep state. The first fall-asleep time point and the third fall-asleep time point are earlier than the first time, which may indicate that the user falls asleep after a period of time after getting on bed. The fifth fall-asleep time point is earlier than the first time, which may indicate that the ambient light becomes dark before the user falls asleep. The sixth fall-asleep time point is earlier than the first time, which may indicate that an ambient sound volume decreases before the user falls asleep. The seventh fall-asleep time point is earlier than the first time, which may indicate that the screen of the electronic device like the mobile phone is turned off before the user falls asleep.

With reference to the third aspect, in some embodiments, when both the fifth fall-asleep time point and the sixth fall-asleep time point are earlier than the seventh fall-asleep time point, and the seventh fall-asleep time point is later than the first time, the processing apparatus may determine second time based on the seventh fall-asleep time point, and determine the second time as the fall-asleep time of the user, where the second time is later than the seventh fall-asleep time point.

It can be learned that, when it is detected, based on the activity amount and the heart rate, that the user falls asleep, the user may be in a state of lying quietly on bed and using the mobile phone, that is, the user actually does not fall asleep. In addition, after the user turns off the screen of the mobile phone, the user usually needs a period of time before falling asleep. Therefore, the second time after a screen of the electronic device like the mobile phone is turned off may be used as the fall-asleep time of the user. Sleep monitoring is performed with reference to the usage of the electronic device like the mobile phone, thereby improving accuracy of detecting the fall-asleep time.

With reference to the third aspect, in some embodiments, when both the sixth fall-asleep time point and the seventh fall-asleep time point are earlier than the fifth fall-asleep time point, the fifth fall-asleep time point is later than the first time, and a time difference between the fifth fall-asleep time point and the first time is less than or equal to a first difference, the processing apparatus may determine third time based on the fifth fall-asleep time point, and determine the third time as the fall-asleep time of the user, where the third time is later than the fifth fall-asleep time point.

It can be learned that, when both the sixth fall-asleep time point and the seventh fall-asleep time point are earlier than the fifth fall-asleep time point, the user may first turn off audio that is being played in a sleep environment and turn off the screen of the electronic device like the mobile phone before turning off a light to sleep. When the fifth fall-asleep time point is later than the first time, and the time difference between the fifth fall-asleep time point and the first time is less than or equal to the first difference, the user may turn off a light and prepare to fall asleep after lying quietly on bed for a period of time in a light-on state. Therefore, the actual fall-asleep time of the user may be slightly later than time when the ambient light brightness becomes dark. Sleep monitoring is performed with reference to the ambient light brightness, thereby improving accuracy of detecting the fall-asleep time.

With reference to the third aspect, in some embodiments, when both the fifth fall-asleep time point and the seventh fall-asleep time point are earlier than the sixth fall-asleep time point, the sixth fall-asleep time point is later than the first time, and a time difference between the sixth fall-asleep time point and the first time is less than or equal to a second difference, the processing apparatus may determine fourth time based on the sixth fall-asleep time point, and determine the fourth time as the fall-asleep time of the user, where the fourth time is later than the sixth fall-asleep time point.

It can be learned that, when both the fifth fall-asleep time point and the seventh fall-asleep time point are earlier than the sixth fall-asleep time point, the user may first turn off the screen of the mobile phone and turn off the light before the audio that is being played is turned off. When the sixth fall-asleep time point is later than the first time, and the time difference between the sixth fall-asleep time point and the first time is less than or equal to the second difference, the user may actively turn off the played audio after lying quietly on bed and listening to music for a period of time. Therefore, the actual fall-asleep time of the user may be slightly later than the sixth fall-asleep time point (namely, time when the ambient sound volume decreases).

With reference to the third aspect, in some embodiments, when the sixth fall-asleep time point is later than the first time, the time difference between the sixth fall-asleep time point and the first time is greater than the second difference, and both the fifth fall-asleep time point and the seventh fall-asleep time point are earlier than the first time, the processing apparatus may determine fifth time based on the first time, and determine the fifth time as the fall-asleep time of the user, where the fifth time is later than the first time.

With reference to the third aspect, in some embodiments, when the sixth fall-asleep time point and the seventh fall-asleep time point are later than the first time, the time difference between the sixth fall-asleep time point and the first time is greater than the second difference, and the fifth fall-asleep time point is earlier than the first time, the processing apparatus may determine sixth time based on the seventh fall-asleep time point, and determine the sixth time as the fall-asleep time of the user, where the sixth time is later than the seventh fall-asleep time point.

With reference to the third aspect, in some embodiments, when the fifth fall-asleep time point and the sixth fall-asleep time point are later than the first time, the time difference between the fifth fall-asleep time point and the first time is less than or equal to the first difference, the time difference between the sixth fall-asleep time point and the first time is greater than the second difference, and the seventh fall-asleep time point is earlier than the first time, the processing apparatus may determine seventh time based on the fifth fall-asleep time point, and determine the seventh time as the fall-asleep time of the user, where the seventh time is later than the fifth fall-asleep time point.

With reference to the third aspect, in some embodiments, when the fifth fall-asleep time point and the sixth fall-asleep time point are later than the first time, the time difference between the fifth fall-asleep time point and the first time is greater than the first difference, the time difference between the sixth fall-asleep time point and the first time is greater than the second difference, and the seventh fall-asleep time point is earlier than the first time, the processing apparatus may determine eighth time based on the first time, and determine the eighth time as the fall-asleep time of the user, where the eighth time is later than the first time.

It can be learned that, when the sixth fall-asleep time point is later than the first time and the time difference between the sixth fall-asleep time point and the first time is greater than the second difference, the user may fall asleep in an audio playing process. The audio continues to be played and is not paused after the user falls asleep. A moment (namely, the sixth fall-asleep time point) when the ambient sound volume decreases cannot reflect the actual fall-asleep time of the user. If the fifth fall-asleep time point and/or the seventh fall-asleep time point are/is within a time period from the first time to the sixth fall-asleep time point, the user may actively turn off the light after lying quietly on bed for a period of time and/or actively turn off the screen of the mobile phone after lying quietly on bed and using the mobile phone for a period of time, and continue to listen to the audio. Therefore, when the moment (namely, the sixth fall-asleep time point) at which the ambient sound volume decreases cannot reflect the actual fall-asleep time of the user, the mobile phone may determine the fall-asleep time point based on the fifth fall-asleep time point and/or the seventh fall-asleep time point. In the foregoing embodiment, sleep monitoring is performed with reference to the ambient light brightness, ambient sound volume, and usage of the mobile phone, thereby improving accuracy of detecting the fall-asleep time.

With reference to the third aspect, in some embodiments, the processing apparatus may determine a first time period based on the fall-asleep time of the user, and obtain a first heart rate and first motion data of the user in the first time period; the processing apparatus may determine a second time period based on wake-up time of the user, and obtain a second heart rate and second motion data of the user in the second time period; the processing apparatus may determine a first activity amount, a first walking feature, and a first get-on-bed action feature based on the first motion data; the processing apparatus may determine a second activity amount, a second walking feature, and a first get-off-bed action feature based on the second motion data; and the processing apparatus may determine get-on-bed time and get-off-bed time of the user based on the first heart rate, the second heart rate, the first activity amount, the first walking feature, the first get-on-bed action feature, the second activity amount, the second walking feature, and the first get-off-bed action feature.

The first heart rate, the first motion data, the second heart rate, and the second motion data may be obtained by the processing apparatus from the data collection apparatus.

It can be learned that, in the foregoing method, the get-on-bed time and get-off-bed time of the user are determined with reference to detection of walking features, heart rates, activity amounts, and a get-on-bed/get-off-bed action near the fall-asleep time and the wake-up time. Walking behaviors obviously change when the user gets on bed and gets off bed, and walking features in a get-on-bed phase and a get-off-bed phase are symmetrical. Therefore, the sleep monitoring method performed with reference to the walking features can improve accuracy of detecting the get-on-bed/get-off-bed time.

With reference to the third aspect, in some embodiments, the processing apparatus may determine a first get-on-bed time point based on the first heart rate and the first activity amount; the processing apparatus may determine a first get-off-bed time point based on the second heart rate and the second activity amount; the processing apparatus may determine a second get-on-bed time point and a second get-off-bed time point based on the first walking feature and the second walking feature; the processing apparatus may determine a third get-on-bed time point based on the first get-on-bed action feature; the processing apparatus may determine a third get-off-bed time point based on the first get-off-bed action feature; the processing apparatus may determine the get-on-bed time of the user based on the first get-on-bed time point, the second get-on-bed time point, and the third get-on-bed time point; and the processing apparatus may determine the get-off-bed time of the user based on the first get-off-bed time point, the second get-off-bed time point, and the third get-off-bed time point.

The first get-on-bed time point is time at which the first heart rate decreases to a first heart rate threshold and the first activity amount decreases to a first activity amount threshold, and the first get-off-bed time point is time at which the second heart rate increases to a second heart rate threshold and/or the second activity amount increases to a second activity amount threshold; the second get-on-bed time point is time at which a last walking behavior occurs in the first time period, and the second get-off-bed time point is time at which a first walking behavior occurs in the second time period; and the third get-on-bed time point is time at which the get-on-bed action occurs, and the third get-off-bed time point is time at which a get-off-bed action occurs.

With reference to the third aspect, in some embodiments, when a time difference between any two of the first get-on-bed time point, the second get-on-bed time point, and the third get-on-bed time point is less than a third difference, the processing apparatus may determine, as the get-on-bed time of the user, an average value of the first get-on-bed time point, the second get-on-bed time point, and the third get-on-bed time point, or determine, as the get-on-bed time of the user, any time between earliest time and latest time in the first get-on-bed time point, the second get-on-bed time point, and the third get-on-bed time point; and when a time difference between any two of the first get-off-bed time point, the second get-off-bed time point, and the third get-off-bed time point is less than a fourth difference, the processing apparatus may determine, as the get-off-bed time of the user, an average value of the first get-off-bed time point, the second get-off-bed time point, and the third get-off-bed time point, or determine, as the get-off-bed time of the user, any time between earliest time and latest time in the first get-off-bed time point, the second get-off-bed time point, and the third get-off-bed time point.

With reference to the third aspect, in some embodiments, when a time difference between the second get-on-bed time point and the third get-on-bed time point is less than the third difference, and a time difference between the first get-on-bed time point and the second get-on-bed time point is greater than or equal to the third difference, the processing apparatus may determine, as the get-on-bed time of the user, an average value of the second get-on-bed time point and the third get-on-bed time point, or determine, as the get-on-bed time of the user, any time between the second get-on-bed time point and the third get-on-bed time point; and when a time difference between the second get-off-bed time point and the third get-off-bed time point is less than the fourth difference, and a time difference between the first get-off-bed time point and the second get-off-bed time point is greater than or equal to the fourth difference, the processing apparatus may determine, as the get-off-bed time of the user, an average value of the second get-off-bed time point and the third get-off-bed time point, or determine, as the get-off-bed time of the user, any time between the second get-off-bed time point and the third get-off-bed time point.

The first time period includes a time period before the fall-asleep time of the user, and the second time period includes a time period after the wake-up time of the user.

According to a fourth aspect, this application provides an electronic device. The electronic device includes a memory and a processor. The memory is configured to store a computer program, and the processor is configured to invoke the computer program, to enable the electronic device to perform any possible implementation method according to the first aspect.

According to a fifth aspect, this application provides a computer-readable storage medium, including instructions. When the instructions are run on an electronic device, the electronic device is enabled to perform any possible implementation method according to the first aspect.

According to a sixth aspect, this application provides a computer program product. The computer program product may include computer instructions. When the computer instructions are run on an electronic device, the electronic device is enabled to perform any possible implementation method according to the first aspect.

According to a seventh aspect, this application provides a chip. The chip is used in an electronic device. The chip includes one or more processors. The processor is configured to invoke computer instructions, to enable the electronic device to perform any possible implementation method according to the first aspect.

It may be understood that, the electronic device provided in the fourth aspect, the computer-readable storage medium provided in the fifth aspect, the computer program product provided in the sixth aspect, and the chip provided in the seventh aspect are all configured to perform the method provided in embodiments of this application. Therefore, for beneficial effect that can be achieved by the electronic device provided in the fourth aspect, the computer-readable storage medium provided in the fifth aspect, the computer program product provided in the sixth aspect, and the chip provided in the seventh aspect, refer to the beneficial effect in the corresponding method. Details are not described herein.

### BRIEF DESCRIPTION OF DRAWINGS

FIG. 1 is a diagram of a communication system according to an embodiment of this application;
FIG. 2 is a diagram of a structure of an electronic device 100 according to an embodiment of this application;
FIG. 3 is a diagram of a structure of another electronic device 100 according to an embodiment of this application;
FIG. 4 is a diagram of a method for determining fall-asleep time according to an embodiment of this application;
FIG. 5 is a diagram of a method for determining get-on-bed/get-off-bed time according to an embodiment of this application;
FIG. 6 is a diagram of a sleep detection result according to an embodiment of this application; and
FIG. 7 is a diagram of another communication system according to an embodiment of this application.

### DESCRIPTION OF EMBODIMENTS

The following describes technical solutions in embodiments of this application with reference to accompanying drawings in embodiments of this application. In the descriptions of embodiments of this application, terms used in the following embodiments are merely intended to describe purposes of specific embodiments, but are not intended to limit this application. The terms "one", "the", "the foregoing", "this", and "the one" of singular forms used in this specification and the appended claims of this application are also intended to include expressions such as "one or more", unless otherwise specified in the context clearly. It should be further understood that, in the following embodiments of this application, "at least one" and "one or more" mean one or more (including two). The term "and/or" is used to describe an association relationship between associated objects and represents that three relationships may exist. For example, A and/or B may represent the following cases: Only A exists, both A and B exist, and only B exists, where A and B may be singular or plural. The character "/" generally indicates an "or" relationship between the associated objects.

Reference to "one embodiment", "some embodiments", or the like described in this specification means that one or more embodiments of this application include a specific feature, structure, or characteristic described with reference to the embodiments. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "have", and their variants all mean "include but are not limited to", unless otherwise specifically emphasized in another manner. The term "connection" includes a direct connection and an indirect connection, unless otherwise specified. "First" and "second" are merely intended for a purpose of description, and shall not be understood as an indication or implication of relative importance or implicit indication of a quantity of indicated technical features.

In embodiments of this application, the term "example", "for example", or the like is used to give an example, an illustration, or a description. Any embodiment or design scheme described as an "example" or "for example" in embodiments of this application should not be construed as being more preferred or more advantageous than another embodiment or design scheme. Exactly, use of the term "example", "for example", or the like is intended to present a related concept in a specific manner.

This application provides a sleep monitoring method. Fall-asleep time of a user is determined with reference to data obtained through a plurality of types of detection, such as heart rate detection, activity amount statistics collection, get-on-bed action detection, walking feature detection, ambient light detection, ambient sound detection, and usage detection of an electronic device like a mobile phone and/or a tablet computer, thereby improving accuracy of detecting the fall-asleep time.

In addition, in this application, get-on-bed time and get-off-bed time of the user can be further determined with reference to heart rate data, activity amount data, and a get-on-bed/get-off-bed walking feature and action feature detected by a wearable device. The get-on-bed time and get-off-bed time can help the user better understand a sleep status of the user.

The sleep monitoring method provided in this application may be applied to a communication system 10.

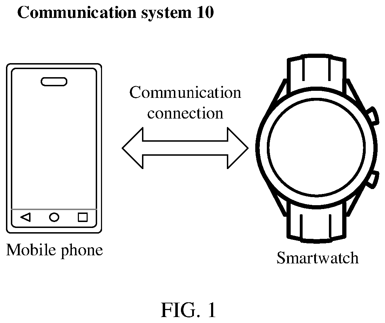

**FIG. 1** **is a diagram of an example of the communication system 10.**

As shown in FIG. 1, the communication system 10 may include a mobile phone and a smartwatch. The mobile phone may establish a communication connection to the smartwatch. For example, the communication connection may be a Bluetooth connection, a wireless local area network (wireless local area network, WLAN) connection, or the like. A manner of the communication connection is not limited in this application.

In some embodiments, the smartwatch may include a heart rate monitoring apparatus, an ambient optical sensor, an audio input apparatus, a motion sensor, and the like. The heart rate monitoring apparatus may be configured to detect heart rate data of a user. For example, the heart rate monitoring apparatus may generate a photoplethysmography (photoplethysmography, PPG) signal, and determine the heart rate data of the user using the PPG signal. The ambient optical sensor may be configured to detect ambient light brightness in an environment in which the smartwatch is located. The audio input apparatus may be configured to capture a sound signal. For example, the audio input apparatus may include a microphone. The motion sensor may be configured to collect motion data, for example, acceleration data and angular velocity data. The motion sensor may include but is not limited to an acceleration sensor, a gyroscope, and the like.

The mobile phone may also include one or more apparatuses such as an ambient optical sensor, an audio input apparatus, and a motion sensor. The mobile phone may detect whether a screen of the mobile phone is turned off. When the mobile phone is in a screen-off state, the mobile phone may further detect whether an audio playing application in the mobile phone is in a running state, that is, whether the mobile phone plays audio in the screen-off state.

In some embodiments, the smartwatch may send one or more pieces of data such as detected heart rate data, brightness data of the ambient light, brightness data of an ambient sound, and motion data to the mobile phone. Then, the mobile phone may determine fall-asleep time of the user based on the data from the smartwatch and usage of the mobile phone. The fall-asleep time may represent time when the user enters sleep from an awake state.

The smartwatch or the mobile phone may further determine wake-up time of the user based on data such as the heart rate data and the motion data detected by the smartwatch. The wake-up time may represent time when the user wakes up.

The smartwatch or the mobile phone may further detect get-on-bed time and get-off-bed time of the user. It may be understood that, the user may not immediately fall asleep after getting on bed, and the user may not immediately get up bed after waking up. Therefore, the get-on-bed time is earlier than the fall-asleep time or is the same as the fall-asleep time, and the get-off-bed time is later than the wake-up time or is the same as the wake-up time. The get-on-bed time may also be referred to as get-up time. The smartwatch or the mobile phone may extract a walking feature and a get-on-bed/get-off-bed action feature from the motion data detected by the smartwatch. Based on the fall-asleep time and the wake-up time, the smartwatch or the mobile phone may determine the get-on-bed time and the get-off-bed time with reference to the walking feature and the get-on-bed/get-off-bed action feature.

In some embodiments, the smartwatch and/or the mobile phone may display sleep data of the user. The sleep data may include but is not limited to the get-on-bed time, the fall-asleep time, the wake-up time, and the get-off-bed time.

In addition to the smartwatch and the mobile phone, the communication system 10 may further include more devices. For example, the communication system 10 may further include wearable devices such as a smart band and smart glasses, a tablet computer, a television, a sound system, and the like. In some embodiments, the mobile phone may establish a communication connection to a device like the wearable device, the tablet computer, the television, or the sound system in the communication system 10. The mobile phone may further determine the sleep data of the user with reference to usage of one or more devices such as the smart glasses, the tablet computer, the television, and the sound system, in addition to the heart rate data, motion data, ambient light brightness data, and ambient sound volume data detected by the smartwatch. For example, the mobile phone may determine, with reference to one or more pieces of data such as a wearing status of the smart glasses, a screen-on/off state of the tablet computer, a screen-on/off state of the television, and an audio playing status of the sound system, whether the user falls asleep. It may be understood that, the smart glasses change from a wearing state to a non-wearing state (that is, the smart glasses are taken off), which may indicate that the user is about to fall asleep. The tablet computer is in the screen-on state, which may indicate that the user is still using the tablet computer, that is, the user has not fallen asleep. The tablet computer enters the screen-off state from the screen-on state, which may indicate that the user is about to fall asleep. Similarly, the television is in the screen-on state, which may indicate that the user is still watching the television, that is, the user has not fallen asleep. The sound system is playing audio, which may indicate that the user is still listening to audio, that is, the user has not fallen asleep.

The following describes a structure of an electronic device in this application.

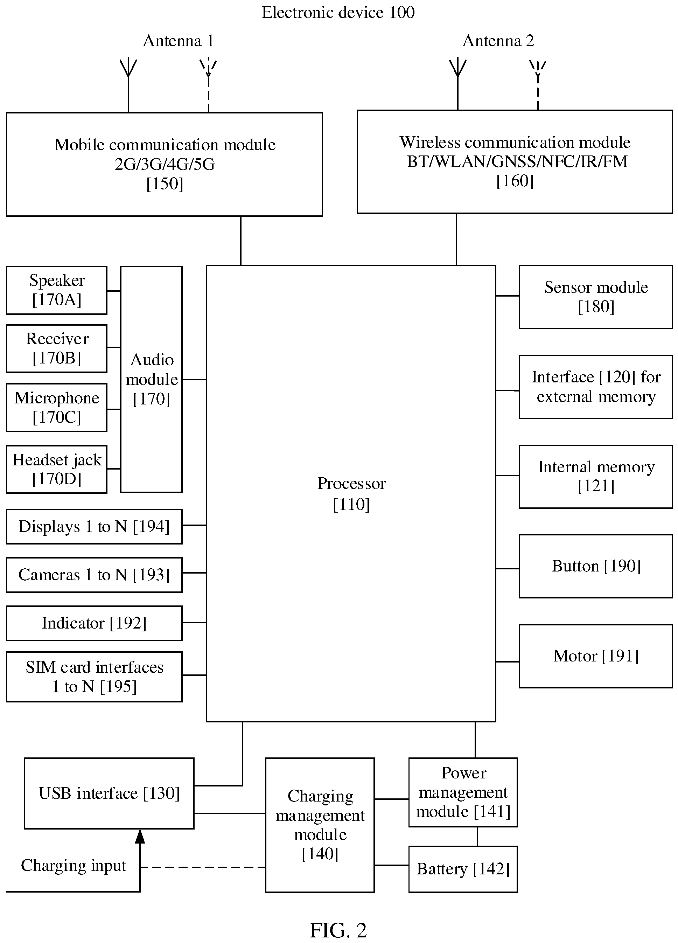

**FIG. 2** **is a diagram of an example of a structure of an electronic device 100.**

As shown in FIG. 2, the electronic device 100 may include a processor 110, an interface 120 for external memory, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identification module (subscriber identification module, SIM) card interface 195, and the like.

It may be understood that, the structure shown in embodiments of this application does not constitute a specific limitation on the electronic device 100. In some other embodiments of this application, the electronic device 100 may include more or fewer components than those shown in the figure, or some components may be combined, or some components may be split, or different component arrangements may be used. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may be a nerve center and a command center of the electronic device 100. The controller may generate an operation control signal based on an instruction operation code and a time sequence signal, to complete control of instruction reading and instruction execution.

The memory may be further disposed in the processor 110, and is configured to store instructions and data. In some embodiments, the memory in the processor 110 is a cache. The memory may store instructions or data just used or cyclically used by the processor 110. If the processor 110 needs to use the instructions or the data again, the processor may directly invoke the instructions or the data from the memory. This avoids repeated access, reduces waiting time of the processor 110, and improves system efficiency.

In this application, the memory may store a computer program, to enable the controller or the processor to implement the sleep monitoring method in this application via an interface or a protocol. For example, the computer program stored in the memory may be used to: detect a heart rate, determine ambient light brightness, determine an ambient sound volume, determine an activity amount based on motion data of a motion sensor, and extract a walking feature and a get-on-bed/get-off-bed action feature from the motion data, determine fall-asleep time, determine wake-up time, determine get-on-bed time, determine get-off-bed time, and the like.

The USB interface 130 is an interface complying with a USB standard. The USB interface 130 may be configured to connect to a charger to charge the electronic device 100, or may be configured to perform data transmission between the electronic device 100 and a peripheral device, or may be configured to connect to a headset for playing audio through the headset.

The charging management module 140 is configured to receive charging input from a charger. The charger may be a wireless charger or a wired charger. The charging management module 140 supplies power to the electronic device via the power management module 141 while charging the battery 142.

The power management module 141 is configured to connect to the battery 142, the charging management module 140, and the processor 110. The power management module 141 receives input of the battery 142 and/or the charging management module 140, to supply power to the processor 110, the internal memory 121, an external memory, the display 194, the camera 193, the wireless communication module 160, and the like.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 150 may provide a wireless communication solution that is applied to the electronic device 100 and that includes 2G/3G/4G/5G or the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1.

The wireless communication module 160 may provide a wireless communication solution that is applied to the electronic device 100 and that includes a WLAN (for example, a wireless fidelity (wireless fidelity, Wi-Fi) network), Bluetooth (Bluetooth, BT), a global navigation satellite system (global navigation satellite system, GNSS), frequency modulation (frequency modulation, FM), near field communication (near field communication, NFC), an infrared (infrared, IR) technology, and the like. The wireless communication module 160 may be one or more components integrating at least one communication processor module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

The electronic device 100 may implement a display function via the GPU, the display 194, the application processor, and the like. The GPU is a microprocessor for image processing, and is connected to the display 194 and the application processor. The GPU is configured to: perform mathematical and geometric computation, and render an image.

The display 194 is configured to display an image, a video, and the like. In some embodiments, the electronic device 100 may include one or N displays 194, where N is a positive integer greater than 1.

The electronic device 100 may implement a photographing function via the ISP, the camera 193, the video codec, the GPU, the display 194, the application processor, and the like.

The ISP is configured to process data fed back by the camera 193. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image.

The camera 193 is configured to capture a static image or a video. In some embodiments, the electronic device 100 may include one or N cameras 193, where N is a positive integer greater than 1.

The digital signal processor is configured to process a digital signal, and may process another digital signal in addition to the digital image signal. For example, when the electronic device 100 selects a frequency, the digital signal processor is configured to perform Fourier transformation on frequency energy.

The NPU is a neural-network (neural-network, NN) computing processor, and quickly processes input information with reference to a structure of a biological neural network, for example, a transfer mode between human brain neurons, and may further continuously perform self-learning. Applications such as intelligent cognition of the electronic device 100 may be implemented through the NPU, for example, image recognition, facial recognition, speech recognition, and text understanding.

The interface 120 for external memory may be used to connect to an external storage card, for example, a micro SD card, to extend a storage capability of the electronic device 100. The external memory card communicates with the processor 110 through the interface 120 for external memory, to implement a data storage function. For example, files such as music and videos are stored in the external storage card.

The internal memory 121 may be configured to store computer-executable program code. The executable program code includes instructions. The processor 110 runs the instructions stored in the internal memory 121, to perform various function applications of the electronic device 100 and data processing. The internal memory 121 may include a program storage area and a data storage area. The program storage area may store an operating system, an application required by at least one function (for example, a sound playing function or an image playing function), and the like. The data storage area may store data (such as audio data or a phone book) created in a use process of the electronic device 100. In addition, the internal memory 121 may include a highspeed random access memory, or may include a non-volatile memory like at least one magnetic disk storage device, a flash memory, or a universal flash storage (universal flash storage, UFS).

The electronic device 100 may implement an audio function, for example, music playing and recording, via the audio module 170, the speaker 170A, the receiver 170B, the microphone 170C, the headset jack 170D, the application processor, and the like.

The audio module 170 is configured to convert digital audio information into an analog audio signal for output, and is also configured to convert analog audio input into a digital audio signal. The audio module 170 may be further configured to encode and decode an audio signal. In some examples, the audio module 170 may be disposed in the processor 110, or some functional modules in the audio module 170 are disposed in the processor 110. The speaker 170A, also referred to as a "loudspeaker", is configured to convert an audio electrical signal into a sound signal. The receiver 170B, also referred to as an "earpiece", is configured to convert an audio electrical signal into a sound signal. The microphone 170C, also referred to as a "mike" or a "mic", is configured to convert a sound signal into an electrical signal. The headset jack 170D is configured to connect to a wired headset.

The speaker 170A and the receiver 170B may belong to an audio output apparatus of the electronic device 100. In addition to the speaker 170A and the receiver 170B, the audio output apparatus of the electronic device 100 may further include another apparatus configured to play audio. The microphone 170C may belong to an audio input apparatus of the electronic device 100. In addition to the microphone 170C, the audio input apparatus of the electronic device 100 may further include another apparatus configured to collect a sound signal.

The sensor module 180 may include a pressure sensor, a gyroscope sensor, a barometric pressure sensor, a magnetic sensor, an acceleration sensor, a gravity sensor, a distance sensor, an optical proximity sensor, a fingerprint sensor, a temperature sensor, a touch sensor, an ambient optical sensor, a bone conduction sensor, and the like.

The gyroscope sensor may be configured to determine a motion posture of the electronic device 100. In some embodiments, angular velocities of the electronic device 100 around three axes (namely, axes x, y, and z) may be determined via the gyroscope sensor.

The acceleration sensor may detect accelerations of the electronic device 100 in various directions (usually on three axes). When the electronic device 100 is still, a magnitude and a direction of gravity may be detected. The acceleration sensor may be further configured to identify a posture of the electronic device, and is used in an application such as switching between a landscape mode and a portrait mode or a pedometer.

In some embodiments, when the electronic device 100 is a device (like a smartwatch or a smart band) worn on a wrist of a user, the electronic device 100 may further detect a posture of an arm of the user via the gyroscope sensor and/or the acceleration sensor. When detecting a sleep status of the user, the electronic device 100 may further use the posture data of the arm of the user.

The button 190 includes a power button, a volume button, and the like. The motor 191 may generate a vibration prompt. The indicator 192 may be indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or removed from the SIM card interface 195, to implement contact with or separation from the electronic device 100. The electronic device 100 may support one or N SIM card interfaces, where N is a positive integer greater than 1. The electronic device 100 interacts with a network through the SIM card, to implement functions such as conversation and data communication. In some examples, the electronic device 100 uses eSIM, namely, an embedded SIM card. The eSIM card may be embedded into the electronic device 100, and cannot be separated from the electronic device 100.

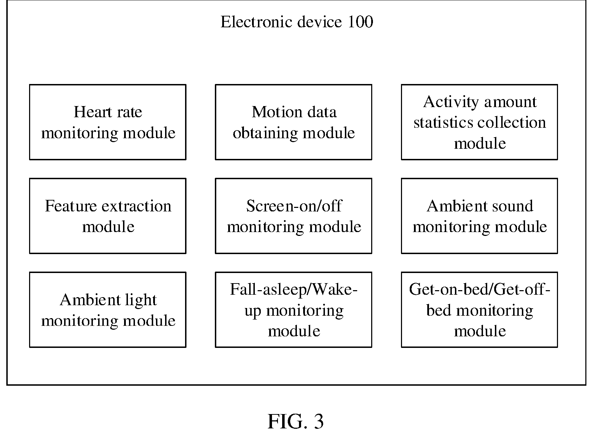

**FIG. 3** **is a diagram of an example of a structure of another electronic device 100.**

As shown in FIG. 3, the electronic device 100 may include a heart rate monitoring module, a motion data obtaining module, an activity amount statistics collection module, a feature extraction module, a screen-on/off monitoring module, an ambient sound monitoring module, an ambient light monitoring module, a fall-asleep/wake-up monitoring module, and a get-on-bed/get-off-bed monitoring module.

The heart rate monitoring module may be configured to detect a heart rate of a user. In some embodiments, the heart rate monitoring module may collect a PPG signal, and determine a heart rate based on the PPG signal. A method for detecting the heart rate is not limited in embodiments of this application.

In some embodiments, the heart rate monitoring module may further determine heart rate variability (heart rate variability, HRV) based on heart rate data. HRV may indicate irregularity of heartbeats. Irregularity of the heartbeats is dominated by an autonomic nerve. Therefore, HRV can reflect health of a nervous system. Higher HRV may indicate a better cardiovascular function and stress resistance. HRV may also reflect an operating status of the autonomic nerve. The autonomic nerve may include a sympathetic nerve for fighting or fleeing, and a parasympathetic nerve for relaxation or digestion. When the user is in a mode dominated by the sympathetic nerve for fighting or fleeing, HRV of the user is low. When the user is in a mode dominated by the parasympathetic nerve for relaxation or digestion, HRV of the user is high.

The electronic device 100 may determine, based on HRV, whether the user falls asleep. In a possible implementation, the electronic device 100 may determine fall-asleep time of the user based on a frequency indicator of HRV. The frequency indicator of the HRV may include high frequency power (high frequency power, HFP) and normalized high frequency power (normalized HFP, nHFP). The HFP may be a variance during a normal heartbeat in a high frequency range, and may represent activity of the parasympathetic nerve. The nHFP may be a quantitative indicator of parasympathetic nerve activity. Time corresponding to a rising edge of the nHFP may be the fall-asleep time of the user.

The motion data obtaining module may be configured to obtain motion data collected by a motion sensor, for example, acceleration data and angular velocity data.

The activity amount statistics collection module may be configured to determine an activity amount based on the motion data. The activity amount may indicate whether the electronic device 100 is moving and whether a posture changes. A faster movement speed of the electronic device 100 indicates a larger posture change, and a larger activity amount. When the electronic device 100 is worn on the user or is held by the user, the activity amount of the electronic device 100 may reflect an activity amount of the user. For example, the electronic device 100 is a smartwatch worn on a wrist of the user. The activity amount determined by the activity amount statistics collection module of the electronic device 100 may reflect an activity status of an arm of the user. A small activity amount determined by the activity amount statistics collection module may indicate that the arm of the user is basically stationary, and a possibility that the user falls asleep is high. A large activity amount determined by the activity amount statistics collection module may indicate that the arm of the user is frequently moving, and a possibility that the user falls asleep is low.

The feature extraction module may be configured to extract a walking feature and an action feature based on the motion data.

The walking feature may include a feature that the user changes from a walking state to a non-walking state, and a feature that the user changes from the non-walking state to the walking state. It may be understood that, the user gets on bed and gets off bed are two opposite processes. A walking feature in a phase in which the user gets on bed and a walking feature in a get-off-bed phase are symmetrical. In a walking process, the user alternately swings through and reaches forward the feet with the two legs. For example, when the left leg is behind and the right leg is in front, the left foot first performs a swing-through action, and then performs a reach-forward action, so that the left and right legs change to a state in which the left leg is in front and the right leg is behind. Then, the right leg first performs a swing-through action, and then performs a reach-forward action, so that the left and right legs change to a state in which the left leg is behind and the right leg is in front again. In the get-on-bed phase, the user changes from the walking state to the non-walking state. The user walks to a bedside, swings through a foot to stop walking, and lies down on bed. Therefore, a get-on-bed walking feature may include an increase in an acceleration in a vertical direction when the user swings through the foot, and a decrease in an acceleration in a horizontal direction when the user swings through the foot. In the get-off-bed phase, the user changes from the non-walking state to the walking state. The user moves to a bedside and reaches forward a foot to start walking. Therefore, a get-off-bed walking feature may include a decrease in an acceleration in a vertical direction when the user reaches forward the foot, and an increase in an acceleration in a horizontal direction when the user reaches forward the foot.

The action feature may include a get-on-bed action feature and a get-off-bed action feature. The action feature may reflect a type of an action performed by the user.

In some embodiments, the feature extraction module may extract the walking feature and the action feature by using a feature extraction algorithm. The feature extraction algorithm may include a principal component analysis (principle component analysis, PCA) algorithm, a support vector machine (support vector machine, SVM) algorithm, and the like. The feature extraction algorithm is not limited in embodiments of this application.

The screen-on/off monitoring module may be configured to detect whether a screen of the electronic device 100 is in a screen-on state or a screen-off state.

The ambient sound monitoring module may be configured to detect a sound volume in an environment in which the electronic device 100 is located. The ambient sound monitoring module may obtain the sound captured by the electronic device 100, and analyze a sound volume.

The ambient light monitoring module may be configured to detect ambient light brightness in the environment in which the electronic device 100 is located. For example, the ambient light monitoring module may include an ambient optical sensor.

The fall-asleep/wake-up monitoring module may be configured to determine the fall-asleep time and wake-up time of the user based on one or more pieces of data such as the heart rate data detected by the heart rate monitoring module, the motion data detected by the motion data obtaining module, the activity amount determined by the activity amount statistics collection module, the walking feature and the action feature determined by the feature extraction module, the screen on/off state determined by the screen on/off detection module, the ambient sound volume determined by the ambient sound monitoring module, and the ambient light brightness determined by the ambient light monitoring module.

In some embodiments, the electronic device 100 may receive data indicating usage of another electronic device. The fall-asleep/wake-up monitoring module of the electronic device 100 may further determine the fall-asleep time and the wake-up time of the user with reference to the data indicating the usage of the another electronic device.

In some embodiments, the electronic device 100 may not include the heart rate monitoring module. The electronic device 100 may obtain heart rate data from another electronic device (for example, a wearable device like a smartwatch). Optionally, when determining the fall-asleep/wake-up time of the user, the electronic device 100 uses one or more pieces of data such as the motion data, ambient sound volume, and ambient light brightness that are detected by another device.

The get-on-bed/get-off-bed monitoring module may be configured to determine get-on-bed time and get-off-bed time of the user based on one or more pieces of data such as the fall-asleep time, the wake-up time, the heart rate data, the activity amount, the walking feature, and the action feature.

A specific method for determining the fall-asleep/wake-up time, and get-on-bed/get-off-bed time of the user is described in a subsequent embodiment. Details are not described herein.

The electronic device 100 is not limited to the modules shown in FIG. 3, and may further include more or fewer modules than those shown in FIG. 3, or combine some modules, or split some modules. The modules shown in FIG. 3 are merely examples for description in this application.

For a structure of an electronic device (like the mobile phone or the smartwatch) in the communication system 10 shown in FIG. 1, refer to the device structures shown in FIG. 2 and FIG. 3.

This application provides a sleep monitoring method. In the method, a heart rate of a user motion data, ambient light brightness, an ambient sound volume, and usage data of one or more electronic devices can be obtained. The motion data may include an acceleration and/or an angular velocity, and the usage data of the one or more electronic devices may include screen-off time of the one or more electronic devices. In the method, an activity amount, a walking feature, and a get-on-bed action feature can be determined based on the motion data, and fall-asleep time of the user can be determined based on the heart rate, the activity amount, the walking feature, the get-on-bed action feature, the ambient light brightness, the ambient sound volume, and the usage data of the one or more electronic devices. The fall-asleep time may also be referred to as a fall-asleep time point.

The one or more electronic devices may include but are not limited to a mobile phone, a tablet computer, a television, a sound system, and a notebook computer. In addition to the screen-off time, the usage data of the one or more electronic devices may further include one or more of the following: duration in which the tablet computer is in a screen-off state, time at which the tablet computer receives a user operation in a screen-on state, time at which the tablet computer plays audio in a screen-off state, duration in which the television plays multimedia content, time at which the television is turned off, duration in which the sound system plays audio, and time at which the sound system stops playing the audio.

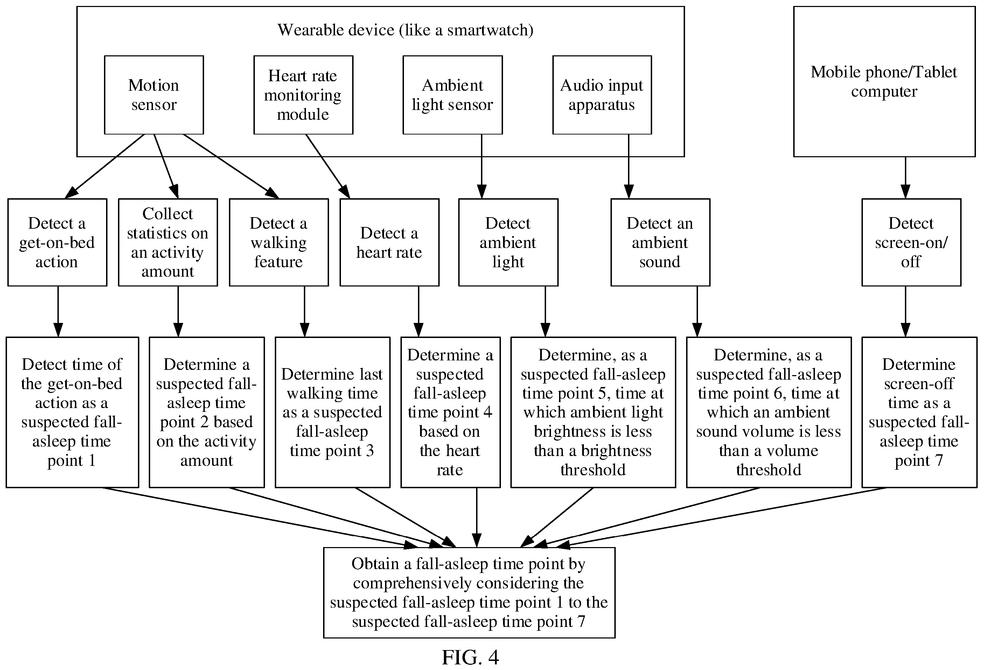

**FIG. 4** **is a diagram of an example of a method for determining fall-asleep time according to this application.**

In some embodiments, in this application, a plurality of suspected fall-asleep time points may be determined based on motion data, heart rate data, ambient light brightness, an ambient sound volume, and a screen-on/off state of a mobile phone and/or a screen-on/off state of a tablet computer that are detected by a wearable device, and then one fall-asleep time point is determined by comprehensively considering the plurality of suspected fall-asleep time points. This fall-asleep time point of a user may be fall-asleep time of the user.

Herein, an example in which a smartwatch and a mobile phone are used to determine the fall-asleep time is used for description.

As shown in FIG. 4, the smartwatch may include a motion sensor, a heart rate monitoring module, an ambient optical sensor, and an audio input apparatus.

The smartwatch may collect motion data such as acceleration data and angular velocity data via the motion sensor. In some embodiments, the smartwatch may detect a get-on-bed action, collect statistics on an activity amount, and detect a walking feature based on the motion data.

The smartwatch may extract an action feature from the motion data by using a feature extraction algorithm like PCA or an SVM. Then, the smartwatch may use the action feature as input of a get-on-bed action monitoring model, and determine the action feature of the get-on-bed action by using the get-on-bed action detection module. The smartwatch may determine occurrence time of the get-on-bed action as a suspected fall-asleep time point 1. The suspected fall-asleep time point 1 may also be referred to as a first fall-asleep time point. The get-on-bed action monitoring model may be a trained neural network model, and may be configured to recognize the feature of the get-on-bed action. A type of the get-on-bed action monitoring model is not limited in embodiments of this application.

The smartwatch may determine the activity amount based on the motion data, and determine a suspected fall-asleep time point 2 based on the activity amount. The suspected fall-asleep time point 2 may also be referred to as a second fall-asleep time point. It may be understood that, after a user falls asleep, the body usually remains stationary or occasionally moves slightly. Therefore, the activity amount detected by the smartwatch after the user falls asleep is small. The smartwatch may determine whether the activity amount in a period of time is less than an activity amount threshold. If the activity amount in the period of time is less than the activity amount threshold, the smartwatch may determine any time point in the period of time as the suspected fall-asleep time point 2. That the activity amount in the period of time is less than the activity amount threshold may include that an average activity amount in the period of time is less than the activity amount threshold. Both the duration of the period of time and the activity amount threshold may be preset. Values of the duration of the period of time and the activity amount threshold are not limited in embodiments of this application.

The smartwatch may further extract the walking feature from the motion data by using the feature extraction algorithm, and determine last walking time as a suspected fall-asleep time point 3 based on the walking feature. The suspected fall-asleep time point 2 may also be referred to as a third fall-asleep time point. The last walking time may include time at which a walking state changes to a non-walking state.

The smartwatch may detect a heart rate of the user via the heart rate monitoring module, and determine a suspected fall-asleep time point 4 based on the heart rate. The suspected fall-asleep time point 4 may also be referred to as a fourth fall-asleep time point. In some embodiments, the smartwatch may determine HRV based on the heart rate, and determine, as the suspected fall-asleep time point 4, time at which a rising edge of an HRV frequency feature nHFP appears.

The smartwatch may detect ambient light brightness via the ambient optical sensor, and determine, as a suspected fall-asleep time point 5, time at which the ambient light brightness is less than a brightness threshold. The suspected fall-asleep time point 5 may also be referred to as a fifth fall-asleep time point. It may be understood that, the user usually turns off a light before falling asleep, and falls asleep in a dark environment. The smartwatch may determine whether ambient light brightness in a period of time is less than the brightness threshold. If the ambient light brightness in the period of time is less than the brightness threshold, the smartwatch may determine any time point in the period of time as the suspected fall-asleep time point 5. That the ambient light brightness in the period of time is less than the brightness threshold may include that an average ambient light brightness value in the period of time is less than the brightness threshold. The brightness threshold may be preset. A value of the brightness threshold is not limited in embodiments of this application.

The smartwatch may collect an ambient sound via the audio input apparatus, and detect an ambient sound volume. The smartwatch may determine, as a suspected fall-asleep time point 6, time at which the ambient sound volume is less than a volume threshold. The suspected fall-asleep time point 6 may also be referred to as a sixth fall-asleep time point. It may be understood that, a sleep environment of the user is usually quiet. The smartwatch may determine whether the ambient sound volume in a period of time is less than the volume threshold. If the ambient sound volume in the period of time is less than the volume threshold, the smartwatch may determine any time point in the period of time as the suspected fall-asleep time point 6. That the ambient sound volume in the period of time is less than the volume threshold may include that an average ambient sound volume value in the period of time is less than the volume threshold. The volume threshold may be preset. A value of the volume threshold is not limited in embodiments of this application.

The mobile phone may detect screen-on/off, and determine whether the mobile phone is in a screen-on state or a screen-off state. It may be understood that, after the user falls asleep, the user will no longer watch a screen of the mobile phone, and the mobile phone remains in the screen-off state for a long time. After detecting that the mobile phone enters the screen-off state, the mobile phone may determine whether the mobile phone is always in the screen-off state in preset duration. If the mobile phone is always in the screen-off state in the preset duration, the mobile phone may determine, as a suspected fall-asleep time point 7, time at which the mobile phone enters the screen-off state (namely, screen-off time). The suspected fall-asleep time point 7 may also be referred to as a seventh fall-asleep time point.

Optionally, the mobile phone may further determine the suspected fall-asleep time point 7 with reference to a user operation on the mobile phone. For example, when the user is ready to fall asleep without turning off a screen of the mobile phone, the mobile phone may not automatically become off-screen, but may remain in a screen-on state. In the screen-on state, the mobile phone may detect whether there is the user operation performed on the mobile phone in a preset time period. The preset time period may be 1 minute, 2 minutes, or the like. Duration of the preset time period is not limited in embodiments of this application. If the mobile phone does not detect the user operation in the preset time period in the screen-on state, the mobile phone may determine any time in the preset time period as the suspected fall-asleep time point 7.

In some embodiments, one or more of the suspected fall-asleep time point 1 to the suspected fall-asleep time point 7 may be determined by the mobile phone. For example, the smartwatch may send, to the mobile phone, the motion data detected by the motion sensor. The mobile phone may detect the get-on-bed action, collect statistics on the activity amount, and detect the walking feature based on the motion data, to determine the suspected fall-asleep time point 1 to the suspected fall-asleep time point 3. The smartwatch may send, to the mobile phone, the heart rate detected by the heart rate monitoring module. The mobile phone may determine the suspected fall-asleep time point 4 based on the heart rate. The smartwatch may send, to the mobile phone, ambient light data detected by the ambient optical sensor. The mobile phone may determine the suspected fall-asleep time point 5 based on the ambient light data from the smartwatch. Optionally, the mobile phone has an ambient optical sensor. The mobile phone may determine the suspected fall-asleep time point 5 based on ambient light data detected by the ambient optical sensor of the mobile phone. The smartwatch may send, to the mobile phone, the ambient sound volume collected via the audio input apparatus. The mobile phone may determine the suspected fall-asleep time point 6 based on the ambient sound volume from the smartwatch. Optionally, the mobile phone has an audio input apparatus. The mobile phone may capture an ambient sound via the audio input apparatus of the mobile phone, and then determine the suspected fall-asleep time point 6 based on an ambient sound volume.

The mobile phone may obtain a fall-asleep time point by comprehensively considering the suspected fall-asleep time point 1 to the suspected fall-asleep time point 7.

In some embodiments, the mobile phone may determine the fall-asleep time point by using, as a reference, the suspected fall-asleep time point 2 determined based on the activity amount and the suspected fall-asleep time point 4 determined based on the heart rate, and using the suspected fall-asleep time point 1, the suspected fall-asleep time point 3, and the suspected fall-asleep time point 5 to the suspected fall-asleep time point 7 as auxiliary. It may be understood that, the activity amount and the heart rate can directly reflect whether the user falls asleep. The get-on-bed action, walking feature, ambient light brightness, ambient sound volume, and screen-off of the mobile phone may reflect that the user is preparing to fall asleep. The user may need a period of time from preparing to fall asleep to actually entering a sleep state. For example, after getting on bed, the user may use the mobile phone for a while before falling asleep. After turning off a light, the user may meditate for a period of time before falling asleep.

After the user falls asleep, the activity amount decreases, and the heart rate also decreases to a value close to a resting heart rate. If an activity amount of the user is high (for example, the user frequently turns over or waves an arm), a heart rate of the user is also high. Therefore, the suspected fall-asleep time point 2 and the suspected fall-asleep time point 4 are usually the same or have a small difference. The mobile phone may detect whether a difference between the suspected fall-asleep time point 2 and the suspected fall-asleep time point 4 is less than a first preset time difference. For example, the first preset time difference may be 2 minutes, 5 minutes, or the like. A value of the first preset time difference is not limited in this application. If the difference between the suspected fall-asleep time point 2 and the suspected fall-asleep time point 4 is less than the first preset time difference, the mobile phone may determine a time point t1 based on the suspected fall-asleep time point 2 and the suspected fall-asleep time point 4. The time point t1 may also be referred to as first time. The time point t1 may be the suspected fall-asleep time point 2, the suspected fall-asleep time point 4, an average value of the suspected fall-asleep time point 2 and the suspected fall-asleep time point 4, or any time point from the suspected fall-asleep time point 2 to the suspected fall-asleep time point 4.

Further, the mobile phone may determine whether the suspected fall-asleep time point 1, the suspected fall-asleep time point 3, and the suspected fall-asleep time point 5 to the suspected fall-asleep time point 7 are earlier than the time point t1 or later than the time point t1. If the suspected fall-asleep time point 1, the suspected fall-asleep time point 3, and the suspected fall-asleep time point 5 to the suspected fall-asleep time point 7 are all earlier than the time point t1, the mobile phone may determine the time point t1 as the fall-asleep time point. It may be understood that, the suspected fall-asleep time point 1 and the suspected fall-asleep time point 3 are earlier than the time point t1, which may indicate that after getting on bed, the user falls asleep after a period of time (namely, a time difference between the suspected fall-asleep time point 1 and the time point t1, or a time difference between the suspected fall-asleep time point 3 and the time point t1). The suspected fall-asleep time point 5 is earlier than the time point t1, which may indicate that the ambient light becomes dark before the user falls asleep. The suspected fall-asleep time point 6 is earlier than the time point t1, which may indicate that an ambient sound volume decreases before the user falls asleep. The suspected fall-asleep time point 7 is earlier than the time point t1, which may indicate that the user turns off the screen of the mobile phone before falling asleep.

If the suspected fall-asleep time point 5 is later than the time point t1, the mobile phone may determine whether a time difference between the suspected fall-asleep time point 5 and the time point t1 is greater than a second preset time difference. The second preset time difference may be 10 minutes, 20 minutes, or the like. A value of the second preset time difference is not limited in embodiments of this application. The time difference between the suspected fall-asleep time point 5 and the time point t1 is greater than the second preset time difference, which may indicate that the user falls asleep in an environment with strong ambient light brightness. For example, the user may fall asleep without turning off the light. Therefore, when the time difference between the suspected fall-asleep time point 5 and the time point t1 is greater than the second preset time difference, the suspected fall-asleep time point 5 may not be used as an evaluation factor for determining the fall-asleep time point.

The time difference between the suspected fall-asleep time point 5 and the time point t1 is less than or equal to the second preset time difference, which may indicate that the ambient light becomes dark in a short period of time after the time point t1. For example, the user turns off the light and prepares to fall asleep after lying quietly on bed for a period of time in a light-on state. When the user lies quietly on bed in the light-on state, the activity amount is small, and the heart rate is slow. The suspected fall-asleep time point 2 and the suspected fall-asleep time point 4 may be time points during which the user lies quietly on bed in the light-on state. Therefore, when the time difference between the suspected fall-asleep time point 5 and the time point t1 is less than or equal to the second preset time difference, the actual fall-asleep time of the user is later than the time point t1 and is close to the suspected fall-asleep time point 5.

If the suspected fall-asleep time point 6 is later than the time point t1, the mobile phone may determine whether a time difference between the suspected fall-asleep time point 6 and the time point t1 is greater than a third preset time difference. The third preset time difference may be 10 minutes, 20 minutes, or the like. A value of the third preset time difference is not limited in embodiments of this application. The time difference between the suspected fall-asleep time point 6 and the time point t1 is greater than the third preset time difference, which may indicate that the user falls asleep in an environment with a large ambient sound volume. For example, the user may start to play music before falling asleep, and fall asleep in a music playing process. The music continues to be played and is not paused after the user falls asleep. The user may be lying quietly on bed, listening to music, and preparing to fall asleep. When the user has not actually fallen asleep, a user status reflected by the activity amount and the heart rate may already be a fall-asleep state. In other words, the suspected fall-asleep time point 2 and the suspected fall-asleep time point 4 may be time points before the user actually falls asleep. Therefore, when the time difference between the suspected fall-asleep time point 6 and the time point t1 is greater than the third preset time difference, the actual fall-asleep time of the user is slightly later than the time point t1.

The time difference between the suspected fall-asleep time point 6 and the time point t1 is less than or equal to the third preset time difference, which may indicate that the ambient sound volume decreases in a short period of time after the time point t1. For example, the user actively turns off the music after lying quietly on bed and listening to the music for a period of time, and then prepares to fall asleep. When the user lies quietly on bed and listens to music, the activity amount is small, and the heart rate is slow. The suspected fall-asleep time point 2 and the suspected fall-asleep time point 4 may be time points during which the user is lying quietly on bed and listens to music and has not fallen asleep yet. Therefore, when the time difference between the suspected fall-asleep time point 6 and the time point t1 is less than or equal to the third preset time difference, the actual fall-asleep time of the user is later than the time point t1 and is close to the suspected fall-asleep time point 6.

The suspected fall-asleep time point 7 is later than the time point t1, which may indicate that the user is still using the mobile phone after the time point t1. For example, the user lies quietly on bed and uses the mobile phone. In a process in which the user lies quietly on bed and uses the mobile phone, the activity amount is small, and the heart rate is slow. Therefore, the suspected fall-asleep time point 2 and the suspected fall-asleep time point 4 may be time points during which the user is lying quietly on bed and using the mobile phone. The actual fall-asleep time of the user is later than the time point t1 and is close to the suspected fall-asleep time point 7.

The suspected fall-asleep time point 1 and the suspected fall-asleep time point 3 are later than the time point t1, which may indicate that the user gets up after falling asleep and gets on bed again. For example, after falling asleep, the user may get up to go to a toilet or drink water, and then get on bed to continue sleeping. The suspected fall-asleep time point 1 and the suspected fall-asleep time point 3 are later than the time point t1, which may not affect the mobile phone in determining the actual fall-asleep time of the user.

In some embodiments, when both the suspected fall-asleep time point 5 and the suspected fall-asleep time point 6 are earlier than the suspected fall-asleep time point 7, and the suspected fall-asleep time point 7 is later than the time point t1, the mobile phone may determine a time point t2 based on the suspected fall-asleep time point 7. The time point t2 may also be referred to as second time. The time point t2 is later than the suspected fall-asleep time point 7. For example, the time point t2 may be a time point later than the suspected fall-asleep time point 7 by a preset time period. For example, the preset time period may be 3 minutes, 5 minutes, 10 minutes, or the like. The mobile phone may determine the time point t2 as the fall-asleep time point.

It may be understood that, when the mobile phone detects, based on the activity amount and the heart rate, that the user falls asleep, the user may be in a state of lying quietly on bed and using the mobile phone, that is, the user actually does not fall asleep. In addition, after the user turns off the screen of the mobile phone, the user usually needs a period of time before falling asleep. Therefore, the mobile phone determines, as the fall-asleep time point, the time point t2 later than time when the screen of the mobile phone is turned off. Sleep monitoring is performed with reference to the usage of the mobile phone, thereby improving accuracy of detecting the fall-asleep time.

In some embodiments, when both the suspected fall-asleep time point 6 and the suspected fall-asleep time point 7 are earlier than the suspected fall-asleep time point 5, the suspected fall-asleep time point 5 is later than the time point t1, and the time difference between the suspected fall-asleep time point 5 and the time point t1 is less than or equal to the second preset time difference, the mobile phone may determine a time point t3 based on the suspected fall-asleep time point 5. The second preset time difference may also be referred to as a first difference. The time point t3 may also be referred to as third time. The time point t3 is later than the suspected fall-asleep time point 5. For example, the time point t3 may be a time point later than the suspected fall-asleep time point 5 by a preset time period. The mobile phone may determine the time point t3 as the fall-asleep time point.

It may be understood that, when both the suspected fall-asleep time point 6 and the suspected fall-asleep time point 7 are earlier than the suspected fall-asleep time point 5, the user may first turn off the audio that is being played in a sleep environment and turn off the screen of the mobile phone before turning off the light to sleep. When the suspected fall-asleep time point 5 is later than the time point t1 and the time difference between the suspected fall-asleep time point 5 and the time point t1 is less than or equal to the second preset time difference, the user may turn off the light and prepare to fall asleep after lying quietly on bed for a period of time in the light-on state. Therefore, the actual fall-asleep time of the user may be slightly later than time when the ambient light brightness becomes dark. Sleep monitoring is performed with reference to the ambient light brightness, thereby improving accuracy of detecting the fall-asleep time.

In some embodiments, when both the suspected fall-asleep time point 5 and the suspected fall-asleep time point 7 are earlier than the suspected fall-asleep time point 6, the suspected fall-asleep time point 6 is later than the time point t1, and the time difference between the suspected fall-asleep time point 6 and the time point t1 is less than or equal to the third preset time difference, the mobile phone may determine a time point t4 based on the suspected fall-asleep time point 6. The third preset time difference may also be referred to as a second difference. The time point t4 may also be referred to as fourth time. The time point t4 is later than the suspected fall-asleep time point 6. For example, the time point t4 may be a time point later than the suspected fall-asleep time point 6 by a preset time period. The mobile phone may determine the time point t4 as the fall-asleep time point.

It may be understood that, when both the suspected fall-asleep time point 5 and the suspected fall-asleep time point 7 are earlier than the suspected fall-asleep time point 6, the user may first turn off the screen of the mobile phone and turn off the light before the audio that is being played is turned off. When the suspected fall-asleep time point 6 is later than the time point t1 and the time difference between the suspected fall-asleep time point 6 and the time point t1 is less than or equal to the third preset time difference, the user may actively turn off the played audio after lying quietly on bed and listening to music for a period of time. Therefore, the actual fall-asleep time of the user may be slightly later than the suspected fall-asleep time point 6 (namely, time when the ambient sound volume decreases).

When the suspected fall-asleep time point 6 is later than the time point t1 and the time difference between the suspected fall-asleep time point 6 and the time point t1 is greater than the third preset time difference, the mobile phone may determine whether the suspected fall-asleep time point 5 and the suspected fall-asleep time point 7 are earlier than the time point t1.

If both the suspected fall-asleep time point 5 and the suspected fall-asleep time point 7 are earlier than the time point t1, the mobile phone may determine a time point t5 based on the time point t1. The time point t5 may also be referred to as fifth time. The time point t5 is later than the time point t1. For example, the time point t5 may be a time point later than the time point t1 by a preset time period. The mobile phone may determine the time point t5 as the fall-asleep time point. It may be understood that, both the suspected fall-asleep time point 5 and the suspected fall-asleep time point 7 are earlier than the time point t1, which may indicate that the user first turns off the light and turns off the screen of the mobile phone before lying quietly on bed to prepare to fall asleep. However, when the user lies quietly on bed, the user may be listening to audio. Therefore, the mobile phone can determine the fall-asleep time of the user with reference to the ambient sound volume, thereby improving accuracy of detecting the fall-asleep time.

If the suspected fall-asleep time point 5 is earlier than the time point t1, and the suspected fall-asleep time point 7 is later than the time point t1, the mobile phone may determine the fall-asleep time point based on the suspected fall-asleep time point 7. For example, the fall-asleep time point determined by the mobile phone based on the suspected fall-asleep time point 7 may be sixth time. The sixth time is later than the suspected fall-asleep time point 7. The fall-asleep time point (for example, the sixth time) determined based on the suspected fall-asleep time point 7 may be a time point later than the suspected fall-asleep time point 7 by a preset time period.

If the suspected fall-asleep time point 5 is later than the time point t1, and the suspected fall-asleep time point 7 is earlier than the time point t1, the mobile phone may determine the fall-asleep time point based on the suspected fall-asleep time point 5 when the time difference between the suspected fall-asleep time point 5 and the time point t1 is less than or equal to the second preset time difference. For example, the fall-asleep time point determined by the mobile phone based on the suspected fall-asleep time point 5 may be seventh time. The seventh time is later than the suspected fall-asleep time point 5. The fall-asleep time point (for example, the seventh time) determined based on the suspected fall-asleep time point 5 may be a time point later than the suspected fall-asleep time point 5 by a preset time period. If the suspected fall-asleep time point 5 is later than the time point t1, and the suspected fall-asleep time point 7 is earlier than the time point t1, the mobile phone may use, when the time difference between the suspected fall-asleep time point 5 and the time point t1 is greater than the second preset time difference, as the fall-asleep time point, the time point t5 determined based on the time point t1. Alternatively, the fall-asleep time point determined by the mobile phone based on the time point t1 is eighth time. The eighth time is later than the time point t1. The eighth time may be the same as or different from the time point t5.

If both the suspected fall-asleep time point 5 and the suspected fall-asleep time point 7 are later than the time point t1, the mobile phone may determine the fall-asleep time point based on an early-late sequence of the suspected fall-asleep time point 5 and the suspected fall-asleep time point 7. For details, refer to the descriptions in the foregoing embodiments. Details are not described herein.

It may be understood that, when the suspected fall-asleep time point 6 is later than the time point t1 and the time difference between the suspected fall-asleep time point 6 and the time point t1 is greater than the third preset time difference, the user may fall asleep in an audio playing process. The audio continues to be played and is not paused after the user falls asleep. A moment (namely, the suspected fall-asleep time point 6) when the ambient sound volume decreases cannot reflect the actual fall-asleep time of the user. If the suspected fall-asleep time point 5 and/or the suspected fall-asleep time point 7 are/is within a time period from the time point t1 to the suspected fall-asleep time point 6, the user may actively turn off the light after lying quietly on bed for a period of time and/or actively turn off the screen of the mobile phone after lying quietly on bed and using the mobile phone for a period of time, and continue to listen to the audio. Therefore, when the moment (namely, the suspected fall-asleep time point 6) at which the ambient sound volume decreases cannot reflect the actual fall-asleep time of the user, the mobile phone may determine the fall-asleep time point based on the suspected fall-asleep time point 5 and/or the suspected fall-asleep time point 7. In the foregoing embodiment, sleep monitoring is performed with reference to the ambient light brightness, ambient sound volume, and usage of the mobile phone, thereby improving accuracy of detecting the fall-asleep time.

The method for determining the fall-asleep time point by comprehensively considering the suspected fall-asleep time point 1 to the suspected fall-asleep time point 7 is merely an example for description in this application, and should not constitute a limitation on this application. The smartwatch and/or the mobile phone may further determine the fall-asleep time point based on the suspected fall-asleep time point 1 to the suspected fall-asleep time point 7 by using another method.

The smartwatch and/or the mobile phone may further generate more or fewer suspected fall-asleep time points, in addition to the suspected fall-asleep time point 1 to the suspected fall-asleep time point 7, and further determine the fall-asleep time point based on all the suspected fall-asleep time points, thereby improving accuracy of detecting the fall-asleep time.

For example, the mobile phone may further obtain usage of a tablet computer, and determine a suspected fall-asleep time point based on the usage of the tablet computer. The usage of the tablet computer may include but is not limited to time at which the tablet computer is turned off, duration in which a screen is in a screen-off state, time at which the tablet computer receives a user operation in a screen-on state, and time at which the tablet computer plays audio in a screen-off state. The suspected fall-asleep time point determined based on the usage of the tablet computer may be a time point at which the user stops using the tablet computer. It may be understood that, when the user is using the tablet computer, it may indicate that the user has not fallen asleep. If the user stops using the tablet computer, the user may be about to fall asleep. To be specific, the actual fall-asleep time of the user is usually later than the suspected fall-asleep time point determined based on the usage of the tablet computer.

The mobile phone may further obtain usage of a television, and determine a suspected fall-asleep time point based on the usage of the television. The usage of the television may include but is not limited to duration in which the television plays multimedia content, and time at which the television is turned off. The suspected fall-asleep time point determined based on the usage of the television may be a time point at which the user stops watching the television. It may be understood that, when the time at which the television is turned off is slightly later than the suspected fall-asleep time point determined based on the activity amount and the heart rate, the user may actively turn off the television after quietly lying on bed and watching the television for a period of time. When the time at which the television is turned off is much later than the suspected fall-asleep time point determined based on the activity amount and the heart rate, the user may fall asleep during watching the television. After the user falls asleep, the television continues playing and is not turned off.

The mobile phone may further obtain usage of a sound system, and determine a suspected fall-asleep time point based on the usage of the sound system. The usage of the sound system may include but is not limited to duration in which the sound system plays audio, and time at which the sound system stops playing the audio. The suspected fall-asleep time point determined based on the usage of the sound system may be a time point at which the user stops listening to the audio.

In some embodiments, the electronic devices such as the smartwatch, the mobile phone, the tablet computer, the television, and the sound system may all be electronic devices associated with the same user. A method for associating the electronic devices with the user is not limited in embodiments of this application. For example, that two electronic devices are associated with a same user may indicate that device accounts logged in to the two electronic devices are accounts of the same user.

In some embodiments, the smartwatch and/or the mobile phone may further detect wake-up time of the user. The smartwatch may determine the wake-up time of the user based on the activity amount and the heart rate. Optionally, based on the activity amount and the heart rate, the mobile phone may further determine the wake-up time of the user with reference to data such as an alarm clock set in the mobile phone and time at which the screen of the mobile phone is turned on and the mobile phone is started to be used by the user. A method for detecting the wake-up time is not limited in embodiments of this application.

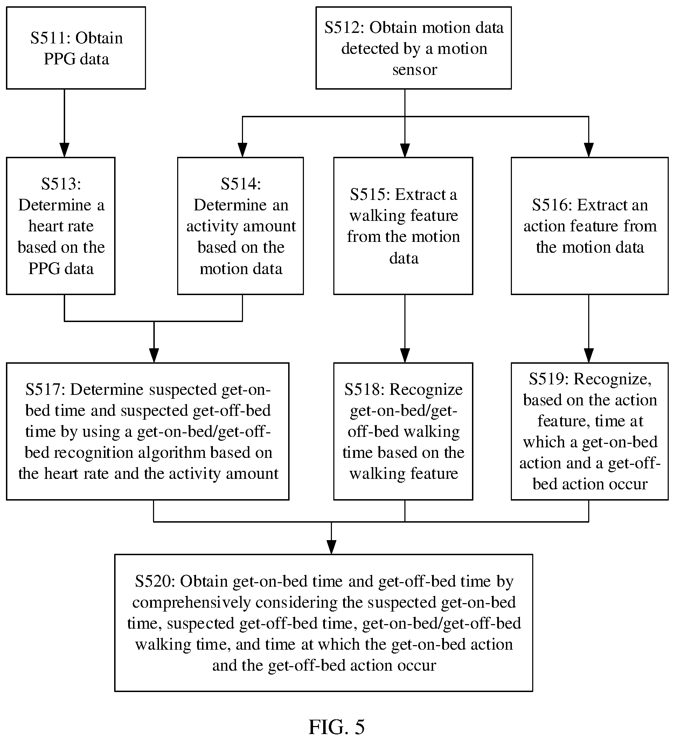

**FIG. 5** **is a diagram of an example of a method for determining get-on-bed/get-off-bed time according to this application.**

In some embodiments, in this application, the get-on-bed time and the get-off-bed time of the user may be determined based on the fall-asleep time, the wake-up time, and the heart rate data and the motion data detected by the wearable device.

Herein, an example in which a smartwatch and a mobile phone are used to determine the get-on-bed/get-off-bed time is used for description.

As shown in FIG. 5, a method for determining get-on-bed/get-off-bed time may include steps S511 to S520.

S511: Obtain PPG data.

S512: Obtain motion data detected by a motion sensor.

S513: Determine a heart rate based on the PPG data.

In some embodiments, the smartwatch may obtain the PPG data via a heart rate monitoring module, and detect the motion data via the motion sensor. The smartwatch may determine the heart rate of a user based on the PPG data. The smartwatch may send the heart rate and the motion data to the mobile phone, and the mobile phone detects fall-asleep/wake-up time and the get-on-bed/get-off-bed time of the user.

S514: Determine an activity amount based on the motion data.

S515: Extract a walking feature from the motion data.

S516: Extract an action feature from the motion data.

For a method for determining the activity amount and extracting the walking feature and the action feature, refer to the descriptions in the foregoing embodiments.

The mobile phone may determine the fall-asleep time and the wake-up time of the user with reference to data such as the heart rate, the activity amount, the walking feature, and the action feature. For details, refer to the descriptions in the embodiment in FIG. 4.

S517: Determine suspected get-on-bed time and suspected get-off-bed time by using a get-on-bed/get-off-bed recognition algorithm based on the heart rate and the activity amount.

In some embodiments, the mobile phone may obtain a heart rate and an activity amount in a period of time before the fall-asleep time, and a heart rate and an activity amount in a period of time after the wake-up time. Duration of the period of time before the fall-asleep time and duration of the period of time after the wake-up time may be 30 minutes, 40 minutes, 1 hour, or the like. A value of the duration is not limited in embodiments of this application.

Based on the heart rate and the activity amount before the user falls asleep, the mobile phone may determine the suspected get-on-bed time by using the get-on-bed/get-off-bed recognition algorithm. Based on the heart rate and the activity amount after the user wakes up, the mobile phone may determine the suspected get-off-bed time by using the get-on-bed/get-off-bed recognition algorithm. The get-on-bed/get-off-bed recognition algorithm may be used to determine the suspected get-on-bed time and the suspected get-off-bed time according to a principle that both the heart rate and activity amount of the user greatly decrease after the user gets on bed, and both the heart rate and activity amount greatly increase after the user gets off bed.

For example, the suspected get-on-bed time determined by using the get-on-bed/get-off-bed recognition algorithm may be time at which the heart rate starts to decrease to a heart rate threshold 1 and the activity amount starts to decrease to an activity amount threshold 1 before the fall-asleep time. Before the suspected get-on-bed time, a heart rate of the user is higher than the heart rate threshold 1 for most of the time, and/or an activity amount of the user is higher than the activity amount threshold 1 for most of the time. After the suspected get-on-bed time, a heart rate of the user is lower than the heart rate threshold 1 for most of the time, and an activity amount is lower than the activity amount threshold 1 for most of the time. The suspected get-off-bed time determined by using the get-on-bed/get-off-bed recognition algorithm may be time at which the heart rate starts to increase to a heart rate threshold 2 and/or the activity amount starts to increase to an activity amount threshold 2 after the wake-up time. Before the suspected get-off-bed time, a heart rate of the user is lower than the heart rate threshold 2 for most of the time, and an activity amount of the user is lower than the activity amount threshold 2 for most of the time. After the suspected get-off-bed time, a heart rate of the user is higher than the heart rate threshold 2 for most of the time, and/or an activity amount is higher than the activity amount threshold 2 for most of the time. A specific implementation of the get-on-bed/get-off-bed recognition algorithm is not limited in embodiments of this application.

S518: Recognize get-on-bed/get-off-bed walking time based on the walking feature.

The user usually has a walking behavior before getting on bed, and almost does not have a walking behavior after getting on bed. The user usually does not have a walking behavior before getting off bed, but has a walking behavior after getting off bed.

In some embodiments, the mobile phone may obtain a walking feature in a period of time before the fall-asleep time, and a walking feature in a period of time after the wake-up time. Based on a change of walking behaviors of the user when the user gets on/off bed, and according to a principle that a walking feature in a get-on-bed phase and a walking feature in a get-off-bed phase are symmetric, the mobile phone may determine the get-on-bed/get-off-bed walking time of the user. The get-on-bed/get-off-bed walking time may include get-on-bed walking time and get-off-bed walking time. The get-on-bed walking time may indicate time at which a last walking behavior occurs before the fall-asleep time. The get-off-bed walking time may indicate time at which a first walking behavior occurs after the wake-up time.

S519: Recognize, based on the action feature, time at which a get-on-bed action and a get-off-bed action occur.

Most users perform similar actions when getting on bed every day. For example, the get-on-bed action may include but is not limited to sitting on bed after arriving at a bedside, then lifting the two legs and placing them on bed, and finally lying down and covering with a blanket. Most users perform similar actions when getting off bed every day. For example, the get-off-bed action may include but is not limited to first sitting up on bed, then moving to a bedside, moving the two legs under bed, putting on shoes, and standing up. The mobile phone may respectively detect the get-on-bed action and the get-off-bed action of the user by using a pre-trained get-on-bed action monitoring model and a pre-trained get-off-bed action monitoring model. The get-on-bed action monitoring model and the get-off-bed action monitoring model may be neural network models. Types of the get-on-bed action monitoring model and the get-off-bed action monitoring model are not limited in embodiments of this application.

In some embodiments, the mobile phone may obtain an action feature in a period of time before the fall-asleep time, and a walking feature in a period of time after the wake-up time. The mobile phone may use the action feature before the user falls asleep as input of the get-on-bed action monitoring model, and detect the get-on-bed action by using the get-on-bed action monitoring model, to determine time at which the get-on-bed action occurs. The mobile phone may use the action feature after the user wakes up as input of the get-off-bed action monitoring model, and detect the get-off-bed action by using the get-off-bed action monitoring model, to determine time at which the get-off-bed action occurs.

In some embodiments, the get-on-bed action monitoring model and the get-off-bed action monitoring model may further perform self-learning, thereby improving accuracy of detecting the get-on-bed action and the get-off-bed action.

S520: Obtain the get-on-bed time and the get-off-bed time by comprehensively considering the suspected get-on-bed time, suspected get-off-bed time, get-on-bed/get-off-bed walking time, and time at which the get-on-bed action and the get-off-bed action occur.

In some embodiments, when the suspected get-on-bed time, get-on-bed walking time, and time at which the get-on-bed action occurs are close, the mobile phone may use any time of the suspected get-on-bed time, get-on-bed walking time, and time at which the get-on-bed action occurs as the get-on-bed time, or use an average value of the suspected get-on-bed time, get-on-bed walking time, and time at which the get-on-bed action occurs as the get-on-bed time, or select any time between earliest time and latest time in the suspected get-on-bed time, get-on-bed walking time, and time at which the get-on-bed action occurs as the get-on-bed time.

When the suspected get-off-bed time, the get-off-bed walking time, and time at which the get-off-bed action occurs are close, the mobile phone may use any time of the suspected get-off-bed time, the get-off-bed walking time, and time at which the get-off-bed action occurs as the get-off-bed time, or use an average value of the suspected get-off-bed time, the get-off-bed walking time, and time at which the get-off-bed action occurs as the get-off-bed time, or select any time between earliest time and latest time in the suspected get-off-bed time, the get-off-bed walking time, and time at which the get-off-bed action occurs as the get-off-bed time.

The suspected get-on-bed time, get-on-bed walking time, and time at which the get-on-bed action occurs are close, which may indicate that a time difference between every two pieces of time is less than a preset time difference (for example, a third difference). The suspected get-off-bed time, the get-off-bed walking time, and time at which the get-off-bed action occurs are close, which may indicate that a time difference between every two pieces of time is less than a preset time difference (for example, a fourth difference).

If the suspected get-on-bed time is later than the get-on-bed walking time and a time difference between the suspected get-on-bed time and the get-on-bed walking time is greater than a preset time difference, it indicates that the user may have a large activity amount after getting on bed. For example, the user frequently turns over after getting on bed, or performs exercises such as stretching before sleep on bed. As a result, the smartwatch still detects a large activity amount and a high heart rate after the user gets on bed. Therefore, when the suspected get-on-bed time is later than the get-on-bed walking time and the time difference between the suspected get-on-bed time and the get-on-bed walking time is greater than the preset time difference, the suspected get-on-bed time may not be used as an evaluation factor for determining the get-on-bed time. In this case, the mobile phone may determine the get-on-bed time based on the get-on-bed walking time and the time at which the get-on-bed action occurs.

If the suspected get-off-bed time is earlier than the get-off-bed walking time and a time difference between the suspected get-off-bed time and the get-off-bed walking time is greater than a preset time difference, it indicates that the user may not get off bed after waking up and may have a large activity amount on bed. For example, the user may use the mobile phone on bed after waking up, or perform exercises such as morning stretching on bed. As a result, the smartwatch detects a large activity amount and a high heart rate before the user gets off bed. Therefore, if the suspected get-off-bed time is earlier than the get-off-bed walking time and the time difference between the suspected get-off-bed time and the get-off-bed walking time is greater than the preset time difference, the suspected get-off-bed time may not be used as an evaluation factor for determining the get-off-bed time. In this case, the mobile phone may determine the get-off-bed time based on the get-off-bed walking time and the time at which the get-off-bed action occurs.

The mobile phone may further determine the get-on-bed time with reference to an activity amount, heart rate, walking feature, and action feature in a period of time after the fall-asleep time, in addition to an activity amount, heart rate, walking feature, and action feature in a period of time before the fall-asleep time. This can avoid inaccurate detection caused by an excessively small amount of data from getting on bed to falling asleep when the user quickly falls asleep after getting on bed. Similarly, the mobile phone may further determine the get-off-bed time with reference to an activity amount, heart rate, walking feature, and action feature in a period of time before the wake-up time, in addition to an activity amount, heart rate, walking feature, and action feature in a period of time after the wake-up time. This can avoid inaccurate detection caused by an excessively small amount of data from waking up to getting off bed after the user wakes up and gets up immediately.

In some embodiments, if no get-off-bed time is detected based on an activity amount, heart rate, walking feature, and action feature in a preset time period after the wake-up-time, the mobile phone may determine, as the get-off-bed time, the preset time after the wake-up time. Duration of the preset time period after the wake-up time may be 1 hour, 2 hours, or the like. The preset time after the wake-up time may be 1 hour, 2 hours, or the like after the wake-up time. This is not limited in embodiments of this application. That no get-off-bed time is detected based on the activity amount and the heart rate may indicate that both the activity amount and the heart rate of the user remain at a low level after the wake-up time. In other words, the get-on-bed/get-off-bed recognition algorithm cannot determine the suspected get-off-bed time based on the activity amount and the heart rate. That no get-off-bed time is detected based on the walking feature may indicate that the user does not have a walking behavior after the wake-up time. In other words, the mobile phone cannot detect the get-off-bed walking time based on the walking feature. That no get-off-bed time is detected based on the action feature may indicate that the user does not have a get-off-bed action after the wake-up time. In other words, the mobile phone cannot detect the get-off-bed action based on the action feature, and therefore cannot determine the time at which the get-off-bed action occurs.

In some embodiments, one or more of steps S514 to S520 may alternatively be performed by the smartwatch.

It can be learned from the method shown in FIG. 5 that, in this application, the get-on-bed time and the get-off-bed time of the user are determined with reference to detection of walking features, heart rates, activity amounts, and a get-on-bed/get-off-bed action near the fall-asleep time and the wake-up time. Walking behaviors obviously change when the user gets on bed and gets off bed, and walking features in a get-on-bed phase and a get-off-bed phase are symmetrical. Therefore, the sleep monitoring method performed with reference to the walking features can improve accuracy of detecting the get-on-bed/get-off-bed time.

This application provides a sleep monitoring method. In the method, a first time period may be determined based on the fall-asleep time of the user, and a first heart rate and first motion data of the user in the first time period are obtained; and a second time period is determined based on the wake-up time of the user, and a second heart rate and second motion data of the user in the second time period are obtained. In the method, a first activity amount, a first walking feature, and a first get-on-bed action feature are determined based on the first motion data, and a second activity amount, a second walking feature, and a first get-off-bed action feature are determined based on the second motion data. Then, in the method, the get-on-bed time and get-off-bed time of the user are determined based on the first heart rate, the second heart rate, the first activity amount, the first walking feature, the first get-on-bed action feature, the second activity amount, the second walking feature, and the first get-off-bed action feature.

The first time period may include a period of time before the fall-asleep time. The second time period may include a period of time after the wake-up time. Optionally, the first time period may further include a period of time after the fall-asleep time. The second time period may further include a period of time before the wake-up time.

In some embodiments, duration of the first time period and the second time period may be the same.

In some embodiments, the mobile phone may determine a first get-on-bed time point based on the first heart rate and the first activity amount, and determine a first get-off-bed time point based on the second heart rate and the second activity amount. The first get-on-bed time point and the first get-off-bed time point may be determined by using the get-on-bed/get-off-bed recognition algorithm. The first get-on-bed time point may also be referred to as suspected get-on-bed time. The first get-off-bed time point may also be referred to as suspected get-off-bed time. The first get-on-bed time point may be time at which the first heart rate decreases to a first heart rate threshold and the first activity amount decreases to a first activity amount threshold. The first get-off-bed time point may be time at which the second heart rate increases to a second heart rate threshold and/or the second activity amount increases to a second activity amount threshold. The first heart rate threshold may also be referred to as a heart rate threshold 1. The first activity amount threshold may also be referred to as an activity amount threshold 1. The second heart rate threshold may also be referred to as a heart rate threshold 2. The second activity amount threshold may also be referred to as an activity amount threshold 2.

The mobile phone may determine a second get-on-bed time point and a second get-off-bed time point based on the first walking feature and the second walking feature. The second get-on-bed time point and the second get-off-bed time point may be determined according to a principle that a walking feature of the user in the get-on-bed phase and a walking feature of the user in the get-off-bed phase are symmetric. The second get-on-bed time point and the second get-off-bed time point are determined based on time at which symmetric features in the first walking feature and the second walking feature occur. The second get-on-bed time point may also be referred to as get-on-bed walking time. The second get-off-bed time point may also be referred to as get-off-bed walking time. The second get-on-bed time point may be time at which a last walking behavior occurs in the first time period. The second get-off-bed time point may be time at which a first walking behavior occurs in the second time period.

The mobile phone may determine a third get-on-bed time point based on the first get-on-bed action feature, and determine a third get-off-bed time point based on the first get-off-bed action feature. The third get-on-bed time point may be determined by using the get-on-bed action monitoring model. The third get-off-bed time point may be determined by using the get-off-bed action monitoring model. The third get-on-bed time point may be time at which a get-on-bed action occurs. The third get-off-bed time point may be time when a get-off-bed action occurs.

In some embodiments, when a time difference between any two of the first get-on-bed time point, the second get-on-bed time point, and the third get-on-bed time point is less than a third difference, the mobile phone may determine, as the get-on-bed time of the user, an average value of the first get-on-bed time point, the second get-on-bed time point, and the third get-on-bed time point, or determine, as the get-on-bed time of the user, any time between earliest time and latest time of the first get-on-bed time point, the second get-on-bed time point, and the third get-on-bed time point.

In some embodiments, when a time difference between any two of the first get-off-bed time point, the second get-off-bed time point, and the third get-off-bed time point is less than a fourth difference, the mobile phone may determine, as the get-off-bed time of the user, an average value of the first get-off-bed time point, the second get-off-bed time point, and the third get-off-bed time point, or determine, as the get-off-bed time of the user, any time between earliest time and latest time of the first get-off-bed time point, the second get-off-bed time point, and the third get-off-bed time point.

In some embodiments, when a time difference between the second get-on-bed time point and the third get-on-bed time point is less than the third difference, and a time difference between the first get-on-bed time point and the second get-on-bed time point is greater than the third difference, the mobile phone may determine the get-on-bed time of the user based on the second get-on-bed time point and the third get-on-bed time point. For example, the mobile phone may determine, as the get-on-bed time of the user, an average value of the second get-on-bed time point and the third get-on-bed time point, or determine, as the get-on-bed time of the user, any time between the second get-on-bed time point and the third get-on-bed time point. In other words, when the time difference between the first get-on-bed time point and the second get-on-bed time point is excessively large, the first get-on-bed time point may not be used as an evaluation factor for determining the get-on-bed time.

In some embodiments, when a time difference between the second get-off-bed time point and the third get-off-bed time point is less than the fourth difference, and a time difference between the first get-off-bed time point and the second get-off-bed time point is greater than the fourth difference, the mobile phone may determine the get-off-bed time of the user based on the second get-off-bed time point and the third get-off-bed time point. For example, the mobile phone may determine, as the get-off-bed time of the user, an average value of the second get-off-bed time point and the third get-off-bed time point, or determine, as the get-off-bed time of the user, any time between the second get-off-bed time point and the third get-off-bed time point. In other words, when the time difference between the first get-off-bed time point and the second get-off-bed time point is excessively large, the first get-off-bed time point may not be used as an evaluation factor for determining the get-off-bed time.

In some embodiments, when sleep data such as fall-asleep/wake-up time and get-on-bed/get-off-bed time is detected, the smartwatch and/or the mobile phone may display the sleep data. Herein, an example in which the mobile phone displays the sleep data is used for description.

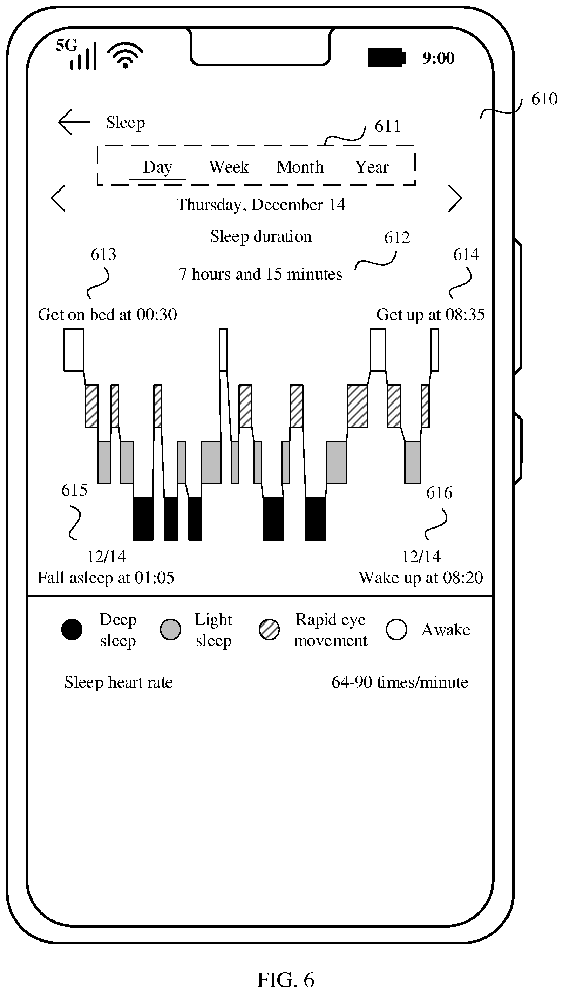

**FIG. 6** **is a diagram of an example of a sleep detection result.**

As shown in FIG. 6, the mobile phone may display a user interface 610. The user interface 610 may include a time option 611, sleep duration 612, get-on-bed time 613, get-off-bed time 614, fall-asleep time 615, and wake-up time 616.

The time option 611 may be used to select to view sleep data in a time period. For example, the time option 611 may include a "day option", a "week option", a "month option", a "year option", and the like. The "day option" may be used to select to view sleep data of one day (for example, yesterday). The "week option" may be used to select to view sleep data of one week (for example, last week). The "month option" may be used to select to view sleep data of one month (for example, last month). The "year option" may be used to select to view sleep data of one year (for example, last year).

The sleep duration 612 may indicate sleep duration of the user, namely, duration from the fall-asleep time to the wake-up time.

The get-on-bed time 613 may indicate the get-on-bed time of the user.

The get-off-bed time 614 may indicate the get-off-bed time of the user.

The fall-asleep time 615 may indicate the fall-asleep time of the user.

The wake-up time 616 may indicate the wake-up time of the user.

In this way, the user can learn of, based on the sleep data, how much time required from getting on bed to falling asleep and how much time required from waking up to getting up, thereby adjusting a sleep habit and improving sleep quality. In addition, the mobile phone can provide a sleep suggestion for the user based on duration from get-on-bed time to fall-asleep time of the user every day and duration from wake-up time to get-off-bed time. For example, when it is detected that the usage of the electronic device like the mobile phone and/or the tablet computer affects the fall-asleep time of the user, the sleep suggestion provided by the mobile phone may include but is not limited to reducing duration of using the electronic device like the mobile phone and/or the tablet computer after getting on bed can help you fall asleep faster. For another example, when it is detected that the ambient light affects the fall-asleep time of the user, the sleep suggestion provided by the mobile phone may include but is not limited to turning off the light as soon as possible after getting on bed can help you fall asleep faster. The sleep suggestion is merely an example for description in this application, and should not constitute a limitation on this application.

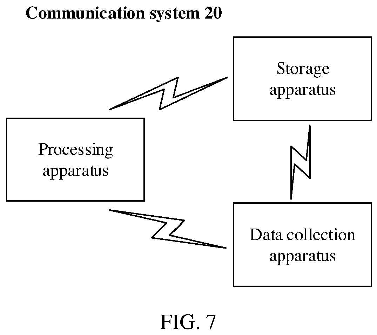

**FIG. 7** **is a diagram of an example of a communication system 20 according to this application.**

The sleep monitoring method provided in this application may be applied to the communication system 20.

As shown in FIG. 7, the communication system 20 may include a processing apparatus, a storage apparatus, and a data collection apparatus.

The data collection apparatus may be configured to collect heart rate data, motion data (such as an acceleration and an angular velocity), ambient light brightness data, ambient sound volume data, and usage data of one or more electronic devices. The usage data of the one or more electronic devices may include screen-off time of screens of the one or more electronic devices.

In some embodiments, the data collection apparatus may include one or more sensors, for example, motion sensors such as an ambient optical sensor, an acceleration sensor, and an angular velocity sensor. The data collection apparatus may further include a heart rate detection apparatus, an audio input apparatus, and the like. Components included in the data collection apparatus may be all configured on one device, or may be configured on a plurality of devices. An existence form of the data collection apparatus is not limited in embodiments of this application. For example, the sensor, the heart rate detection apparatus, and the audio input apparatus included in the data collection apparatus may all be components on a wearable device (for example, a smartwatch). Alternatively, the sensor and the heart rate detection apparatus included in the data collection apparatus may be components on a smartwatch, and the audio input apparatus may be a component on a mobile phone. Alternatively, the heart rate detection apparatus included in the data collection apparatus may be an independent device, and the sensor and the audio input apparatus may be components on a smartwatch.

In some embodiments, the data collection apparatus may send collected data to the processing apparatus. The processing apparatus may determine sleep data such as fall-asleep time, wake-up time, get-on-bed time, and get-off-bed time of the user based on the data collected by the data collection apparatus. The processing apparatus may detect a get-on-bed action, collect statistics on an activity amount, and detect a walking feature based on the motion data. The processing apparatus may further determine the suspected fall-asleep time point 1 to the suspected fall-asleep time point 7 shown in FIG. 4 based on a get-on-bed action, an activity amount, a walking feature, a heart rate, ambient light brightness, an ambient sound volume, and the usage data of the electronic device. Then, the processing apparatus may determine a fall-asleep time point by comprehensively considering the suspected fall-asleep time point 1 to the suspected fall-asleep time point 7. The processing apparatus may further extract the walking feature and an action feature from the motion data, and determine get-on-bed/get-off-bed walking time, and time at which a get-on-bed action and a get-off-bed action occur. The processing apparatus may determine suspected get-on-bed time and suspected get-off-bed time based on the heart rate and the activity amount. Further, the processing apparatus may determine get-on-bed time and get-off-bed time of the user. For a specific process in which the processing apparatus determines the sleep data, refer to the methods shown in FIG. 4 and FIG. 5. Details are not described herein.

In some embodiments, the processing apparatus may include processors configured on one or more devices. An existence form of the processing apparatus is not limited in embodiments of this application. For example, the processing apparatus may include a processor of a smartwatch and a processor of a mobile phone. The processor of the mobile phone may detect the get-on-bed action, collect statistics on the activity amount, and detect the walking feature based on the motion data. The processor of the mobile phone may further determine the suspected fall-asleep time points 1 to 3 and the suspected fall-asleep time point 7 shown in FIG. 4, and obtain the fall-asleep point by comprehensively considering the suspected fall-asleep time point 1 to the suspected fall-asleep time point. The processor of the smartwatch may determine the suspected fall-asleep time point 4 to the suspected fall-asleep time point 6 shown in FIG. 4. Alternatively, the processing apparatus may be an independent apparatus. For example, a processor may be a processor of a mobile phone. The processor of the mobile phone may perform all the foregoing steps of determining the sleep data of the user based on the data collected by the data collection apparatus.

In some embodiments, the data collection apparatus may send the collected data to the storage apparatus. Then, the processor apparatus may obtain, from the storage apparatus, the data collected by the data collection apparatus. The processing apparatus may further send the detected sleep data of the user to the storage apparatus. The storage apparatus may store the data collected by the data collection apparatus, the sleep data detected by the processing apparatus, and the like.

The storage apparatus may further store a computer program used to enable the processing apparatus to detect the sleep data. The processing apparatus may obtain the computer program from the storage apparatus, and then execute the computer program to detect the sleep data.

In some embodiments, the storage apparatus may include memories configured on one or more devices. An existence form of the storage apparatus is not limited in embodiments of this application. For example, the storage apparatus may include a memory of a smartwatch and a memory of a mobile phone. Alternatively, the storage apparatus may include only a memory of a smartwatch, or include only a memory of a mobile phone.

In some embodiments, the communication system 20 may further include more apparatuses. For example, the communication system 20 may further include a display apparatus. The display apparatus may include displays on one or more devices. The display apparatus may be configured to display the sleep data detected by the processing apparatus, for example, the fall-asleep time, the wake-up time, the get-on-bed time, and the get-off-bed time.

It may be understood that, the user interface described in embodiments of this application is merely an example interface, and does not constitute a limitation on the solutions of this application. In another embodiment, the user interface may use different interface layouts, may include more or fewer controls, and may add or reduce other function options, and provided that the user interface is based on a same inventive idea provided in this application, all fall within the protection scope of this application.

It should be noted that, if no contradiction or conflict occurs, any feature or any part of any feature in any embodiment of this application may be combined, and a combined technical solution also falls within the scope of embodiments of this application.

In conclusion, the foregoing embodiments are merely intended for describing the technical solutions of this application, but not for limiting this application. Although this application is described in detail with reference to the foregoing embodiments, persons of ordinary skill in the art should understand that they may still make modifications to the technical solutions described in the foregoing embodiments or make equivalent replacements to some technical features thereof, without departing from the scope of the technical solutions of embodiments of this application.

## Claims

1. A sleep monitoring method, wherein the method comprises:
obtaining a heart rate and motion data of a user, wherein the motion data comprises an acceleration and/or an angular velocity;
obtaining ambient light brightness, an ambient sound volume, and usage data of one or more electronic devices, wherein the usage data of the one or more electronic devices comprises screen-off time of the one or more electronic devices;
determining an activity amount, a walking feature, and a get-on-bed action feature based on the motion data; and
determining fall-asleep time of the user based on the heart rate, the activity amount, the walking feature, the get-on-bed action feature, the ambient light brightness, the ambient sound volume, and the usage data of the one or more electronic devices.

2. The method according to claim 1, wherein determining the fall-asleep time of the user based on the heart rate, the activity amount, the walking feature, the get-on-bed action feature, the ambient light brightness, the ambient sound volume, and the usage data of the one or more electronic devices specifically comprises:
determining a first fall-asleep time point based on the get-on-bed action feature, determining a second fall-asleep time point based on the activity amount, determining a third fall-asleep time point based on the walking feature, determining a fourth fall-asleep time point based on the heart rate, determining a fifth fall-asleep time point based on the ambient light brightness, determining a sixth fall-asleep time point based on the ambient sound volume, and determining a seventh fall-asleep time point based on the usage data of the one or more electronic devices; and
determining the fall-asleep time of the user based on the first fall-asleep time point, the second fall-asleep time point, the third fall-asleep time point, the fourth fall-asleep time point, the fifth fall-asleep time point, the sixth fall-asleep time point, and the seventh fall-asleep time point.

3. The method according to claim 2, wherein the first fall-asleep time point is time at which a get-on-bed action occurs, the second fall-asleep time point is time at which the activity amount is less than an activity amount threshold, the third fall-asleep time point is time at which the user changes from a walking state to a non-walking state, the fifth fall-asleep time point is time at which the ambient light brightness is less than a brightness threshold, the sixth fall-asleep time point is time at which the ambient sound volume is less than a volume threshold, and the seventh fall-asleep time point is time at which the one or more electronic devices become off-screen.

4. The method according to claim 2 or 3, wherein determining the fall-asleep time of the user based on the first fall-asleep time point, the second fall-asleep time point, the third fall-asleep time point, the fourth fall-asleep time point, the fifth fall-asleep time point, the sixth fall-asleep time point, and the seventh fall-asleep time point specifically comprises:
determining first time based on the second fall-asleep time point and the fourth fall-asleep time point; and
when the first fall-asleep time point, the third fall-asleep time point, the fifth fall-asleep time point, the sixth fall-asleep time point, and the seventh fall-asleep time point are all earlier than the first time, determining the first time as the fall-asleep time of the user.

5. The method according to claim 4, wherein the first time is any time between the second fall-asleep time point and the fourth fall-asleep time point, or an average value of the second fall-asleep time point and the fourth fall-asleep time point.

6. The method according to claim 4 or 5, wherein the method further comprises:
when both the fifth fall-asleep time point and the sixth fall-asleep time point are earlier than the seventh fall-asleep time point, and the seventh fall-asleep time point is later than the first time, determining second time based on the seventh fall-asleep time point, and determining the second time as the fall-asleep time of the user, wherein the second time is later than the seventh fall-asleep time point.

7. The method according to any one of claims 4 to 6, wherein the method further comprises:
when both the sixth fall-asleep time point and the seventh fall-asleep time point are earlier than the fifth fall-asleep time point, the fifth fall-asleep time point is later than the first time, and a time difference between the fifth fall-asleep time point and the first time is less than or equal to a first difference, determining third time based on the fifth fall-asleep time point, and determining the third time as the fall-asleep time of the user, wherein the third time is later than the fifth fall-asleep time point.

8. The method according to any one of claims 4 to 7, wherein the method further comprises:
when both the fifth fall-asleep time point and the seventh fall-asleep time point are earlier than the sixth fall-asleep time point, the sixth fall-asleep time point is later than the first time, and a time difference between the sixth fall-asleep time point and the first time is less than or equal to a second difference, determining fourth time based on the sixth fall-asleep time point, and determining the fourth time as the fall-asleep time of the user, wherein the fourth time is later than the sixth fall-asleep time point.

9. The method according to any one of claims 4 to 8, wherein the method further comprises:
when the sixth fall-asleep time point is later than the first time, the time difference between the sixth fall-asleep time point and the first time is greater than the second difference, and both the fifth fall-asleep time point and the seventh fall-asleep time point are earlier than the first time, determining fifth time based on the first time, and determining the fifth time as the fall-asleep time of the user, wherein the fifth time is later than the first time.

10. The method according to any one of claims 4 to 9, wherein the method further comprises:
when the sixth fall-asleep time point and the seventh fall-asleep time point are later than the first time, the time difference between the sixth fall-asleep time point and the first time is greater than the second difference, and the fifth fall-asleep time point is earlier than the first time, determining sixth time based on the seventh fall-asleep time point, and determining the sixth time as the fall-asleep time of the user, wherein the sixth time is later than the seventh fall-asleep time point.

11. The method according to any one of claims 4 to 10, wherein the method further comprises:
when the fifth fall-asleep time point and the sixth fall-asleep time point are later than the first time, the time difference between the fifth fall-asleep time point and the first time is less than or equal to the first difference, the time difference between the sixth fall-asleep time point and the first time is greater than the second difference, and the seventh fall-asleep time point is earlier than the first time, determining seventh time based on the fifth fall-asleep time point, and determining the seventh time as the fall-asleep time of the user, wherein the seventh time is later than the fifth fall-asleep time point.

12. The method according to any one of claims 4 to 11, wherein the method further comprises:
when the fifth fall-asleep time point and the sixth fall-asleep time point are later than the first time, the time difference between the fifth fall-asleep time point and the first time is greater than the first difference, the time difference between the sixth fall-asleep time point and the first time is greater than the second difference, and the seventh fall-asleep time point is earlier than the first time, determining eighth time based on the first time, and determining the eighth time as the fall-asleep time of the user, wherein the eighth time is later than the first time.

13. The method according to any one of claims 1 to 12, wherein the method further comprises:
determining a first time period based on the fall-asleep time of the user, and obtaining a first heart rate and first motion data of the user in the first time period;
determining a second time period based on wake-up time of the user, and obtaining a second heart rate and second motion data of the user in the second time period;
determining a first activity amount, a first walking feature, and a first get-on-bed action feature based on the first motion data, and determining a second activity amount, a second walking feature, and a first get-off-bed action feature based on the second motion data; and
determining get-on-bed time and get-off-bed time of the user based on the first heart rate, the second heart rate, the first activity amount, the first walking feature, the first get-on-bed action feature, the second activity amount, the second walking feature, and the first get-off-bed action feature.

14. The method according to claim 13, wherein determining the get-on-bed time and the get-off-bed time of the user based on the first heart rate, the second heart rate, the first activity amount, the first walking feature, the first get-on-bed action feature, the second activity amount, the second walking feature, and the first get-off-bed action feature specifically comprises:
determining a first get-on-bed time point based on the first heart rate and the first activity amount;
determining a first get-off-bed time point based on the second heart rate and the second activity amount;
determining a second get-on-bed time point and a second get-off-bed time point based on the first walking feature and the second walking feature;
determining a third get-on-bed time point based on the first get-on-bed action feature;
determining a third get-off-bed time point based on the first get-off-bed action feature;
determining the get-on-bed time of the user based on the first get-on-bed time point, the second get-on-bed time point, and the third get-on-bed time point; and
determining the get-off-bed time of the user based on the first get-off-bed time point, the second get-off-bed time point, and the third get-off-bed time point.

15. The method according to claim 14, wherein the first get-on-bed time point is time at which the first heart rate decreases to a first heart rate threshold and the first activity amount decreases to a first activity amount threshold, and the first get-off-bed time point is time at which the second heart rate increases to a second heart rate threshold and/or the second activity amount increases to a second activity amount threshold; the second get-on-bed time point is time at which a last walking behavior occurs in the first time period, and the second get-off-bed time point is time at which a first walking behavior occurs in the second time period; and the third get-on-bed time point is time at which the get-on-bed action occurs, and the third get-off-bed time point is time at which a get-off-bed action occurs.

16. The method according to claim 14 or 15, wherein determining the get-on-bed time of the user based on the first get-on-bed time point, the second get-on-bed time point, and the third get-on-bed time point and determining the get-off-bed time of the user based on the first get-off-bed time point, the second get-off-bed time point, and the third get-off-bed time point specifically comprise:
when a time difference between any two of the first get-on-bed time point, the second get-on-bed time point, and the third get-on-bed time point is less than a third difference, determining, as the get-on-bed time of the user, an average value of the first get-on-bed time point, the second get-on-bed time point, and the third get-on-bed time point, or determining, as the get-on-bed time of the user, any time between earliest time and latest time in the first get-on-bed time point, the second get-on-bed time point, and the third get-on-bed time point; and
when a time difference between any two of the first get-off-bed time point, the second get-off-bed time point, and the third get-off-bed time point is less than a fourth difference, determining, as the get-off-bed time of the user, an average value of the first get-off-bed time point, the second get-off-bed time point, and the third get-off-bed time point, or determining, as the get-off-bed time of the user, any time between earliest time and latest time in the first get-off-bed time point, the second get-off-bed time point, and the third get-off-bed time point.

17. The method according to any one of claims 14 to 16, wherein determining the get-on-bed time of the user based on the first get-on-bed time point, the second get-on-bed time point, and the third get-on-bed time point and determining the get-off-bed time of the user based on the first get-off-bed time point, the second get-off-bed time point, and the third get-off-bed time point specifically comprise:
when a time difference between the second get-on-bed time point and the third get-on-bed time point is less than the third difference, and a time difference between the first get-on-bed time point and the second get-on-bed time point is greater than or equal to the third difference, determining, as the get-on-bed time of the user, an average value of the second get-on-bed time point and the third get-on-bed time point, or determining, as the get-on-bed time of the user, any time between the second get-on-bed time point and the third get-on-bed time point; and
when a time difference between the second get-off-bed time point and the third get-off-bed time point is less than the fourth difference, and a time difference between the first get-off-bed time point and the second get-off-bed time point is greater than or equal to the fourth difference, determining, as the get-off-bed time of the user, an average value of the second get-off-bed time point and the third get-off-bed time point, or determining, as the get-off-bed time of the user, any time between the second get-off-bed time point and the third get-off-bed time point.

18. The method according to any one of claims 13 to 17, wherein the first time period comprises a time period before the fall-asleep time of the user, and the second time period comprises a time period after the wake-up time of the user.

19. The method according to any one of claims 13 to 18, wherein the method further comprises:
displaying the fall-asleep time, wake-up time, get-on-bed time, and get-off-bed time of the user.

20. The method according to any one of claims 1 to 19, wherein the heart rate and motion data of the user are obtained by a wearable device, and the wearable device comprises one or more of the following: a smartwatch and a smart band.

21. The method according to any one of claims 1 to 20, wherein the one or more electronic devices comprise one or more of the following: a mobile phone, a tablet computer, a television, and a notebook computer.

22. An electronic device, wherein the electronic device comprises a memory and a processor, the memory is configured to store a computer program, and the processor is configured to invoke the computer program, to enable the electronic device to perform the method according to any one of claims 1 to 21.

23. A computer-readable storage medium, storing instructions, wherein when the instructions are run on an electronic device, the electronic device is enabled to perform the method according to any one of claims 1 to 21.

24. A computer program product, wherein the computer program product comprises computer instructions, and when the computer instructions are run on an electronic device, the electronic device is enabled to perform the method according to any one of claims 1 to 21.
